# EUROPEAN PATENT APPLICATION

(11) **EP 1 666 009 A2**
(43) Date of publication of application: **07.06.2006**
(21) Application number: 05013025.1
(22) Date of filing: 20.07.2001
(51) Int. Cl.: A61F 9/01

(54) **System for refractive ophthalmic surgery**

(30) Priority: 20.07.2000 US 220019 P
(62) Divisional of application: 01954818.9
(71) Applicant: THE OHIO STATE UNIVERSITY, Columbus, OH 43210-1063 (US)
(72) Inventor: Roberts, Cynthia, Columbus, Ohio 43220 (US); Dupps, William J.Jr, Bay Village, Ohio 44140 (US); Katsube, Noriko, Columbus Ohio 43235 (US)
(74) Representative: Jackson, Nicholas Andrew

(57) **Abstract**

Biomechanical responses of the eye are used to improve photorefractive procedures. LASIK or PRK treatments, for example, can be improved by taking pre-operative measurement and predicting the corneas's biodynamic response to the ablative treatment. Predicitve use is mad of the biodynamic response of the cornea due to laser or mechanical keratectomy, that is, creating a corneal flap characterisitc of LASIK. Comparison of pre-flap and post-flap, (pre-ablation) data of the cornea such as corneal thickness, flap thickness, corneal topography and wavefront, for example, can provide predictive information applicable to modifying an ablation alorithm before the laser is engaged, either for a current operation or the development of a model. Modeling, by finite element analysis or other mathematical techniques, can also be used to predict post-operative outcomes based on a pre-operative (no flap cut or other surgical intervention) data for the cornea that is input for an accurate eye model that, in consideration of biodynamic response <i>via</i> the model, provides predictive information for optimizing the sucess of the refractive surgery and ultimately patient satisfaction.

## Description

### Field of the Invention

The invention relates generally to the field of vision correction and more particularly to methods and apparatus involving biodynamics/biomechanics of the eye to ultimately provide improved vision to patients through refractive correction.

### Background of the Invention

The future of laser refractive surgery is tremendously exciting as we move into the new millennium with the potential for ever-improved post-operative visual performance. In the past, the operative goal has been 20/20 visual acuity with zero residual refractive error. Criteria for a "successful" procedure were operationally broadened to 20/40 or better with ±1 diopter of residual refractive error. However, neither surgeons nor future patients will likely be satisfied with such gross data of visual performance. Hence, considerable research effort is being devoted to develop "customized" procedures for each individual patient The new ultimate operative goal is 20/10 visual acuity with aberration-free post-operative vision. Yet, how can these lofty goals be accomplished? First, lasers have been improved with the development of scanning, small spot systems, as opposed to the first generation broad-beam systems. The scanning systems have brought customized procedures into the realm of feasibility. Second, more comprehensive and sophisticated input data can be used to "guide" the laser based on individual patient data, as opposed to the simple sphere and cylinder of the past. Two types of approaches are currently being pursued - wavefront-guided and topography-guided procedures. Early results are promising, yet neither approach has demonstrated *consistently* superior results to non-guided procedures in controlled, scientific studies. Is a piece of the perfect vision puzzle still missing? Is there an additional, complimentary approach to "customization" that has yet to emerge? Are we leaping into the future ahead of our understanding of how these procedures may be implemented to be optimally successful?

Approximately 60 percent of Americans have refractive errors, and millions of people are myopic worldwide. Many thousands of laser refractive surgeries are performed every year for the correction of myopia. These procedures will ultimately affect a huge number of individuals around the globe, and yet the corneal response to laser ablation is not well understood. Of the many individuals treated, about 15-50% of them do NOT achieve 20/20 vision, which translates into very large numbers when the extreme popularity of refractive surgery is considered. It is crucial that the number of patients who achieve their targeted vision be increased in order to improve the overall quality of vision in this vast group of people.

In the quest for the ideal corneal ablation, a major obstacle yet to be overcome is the inadequacy of current models for predicting the *corneal response* to ablation, which impacts the success of both topography-guided and wavefront-guided customized procedures. In its current state, the process of ablation design relies on a "black box" approach, wherein enormous efforts are directed at linking "input" variables (the ablation algorithm) to "output" variables (refractive error, visual acuity, glare, aberration, patient satisfaction, etc.), while the physical interface between input and output-the actual mechanisms of interaction between all relevant components-is largely neglected. Ablation and surgical outcome are ultimately linked on two levels: a deterministic, cause-effect relationship dictated by physical reality, and a statistical relationship, rooted in probability and defined in retrospective regression analyses of the same variables in large-scale clinical trials. The ability to objectively measure performance of the eye, as opposed to simple corneal shape, is critical to "customizing" ablation algorithms and generating an overall improvement of visual outcomes after refractive surgery. Anterior corneal surface topography cannot take into account contributions of optically important structures inside the eye, such as the posterior corneal surface and the crystalline lens. If a laser were programmed strictly with anterior topography data, the correction would be at best incomplete, and at worst simply wrong. Therefore, wavefront analysis is clearly important, particularly if the ultimate goal is to correct higher order aberrations along with the sphere and cylinder. However, the question must be asked whether wavefront analysis alone is sufficient to fully predict visual outcomes. Will it replace corneal topography in the quest for the perfect "aberration-free"guided procedure? Or, on the other hand, is there a piece of the puzzle still missing? If so, can corneal topography complement the wavefront data to help complete the picture of corneal response?

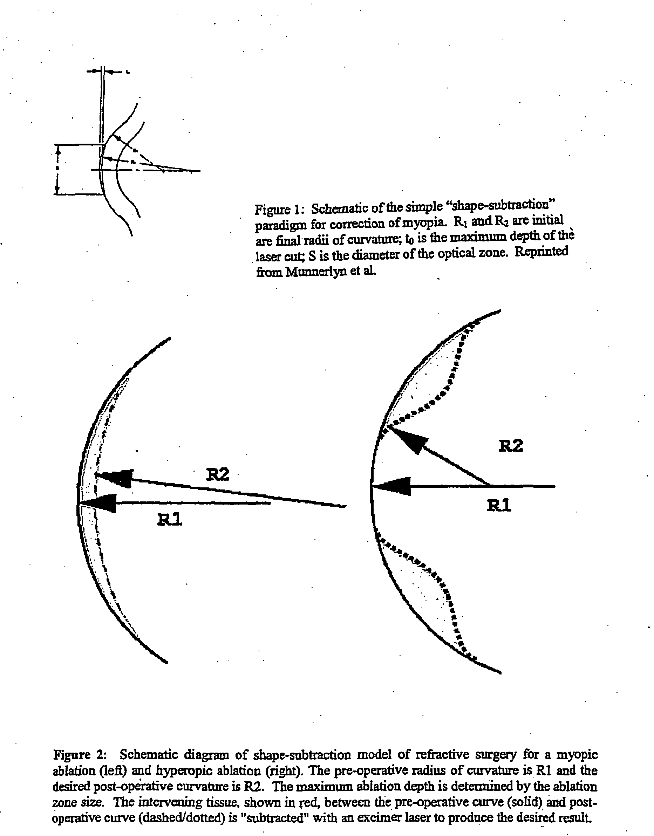

Initial attempts at photorefractive keratectomy (PRK) used a simple model presented by in 1988 by Munnerlyn et al. (Munnerlyn CR, Koons SJ, Marshall J. Photorefractive keratectomy: a technique for laser refractive surgery, *J Cataract Refract Surg.* 1988;14:46-52) The cornea was modeled as two refracting surfaces with a bulk material in between with a known index of refraction. In the treatment of myopia, the goal was to increase the anterior radius of curvature, thus decreasing the curvature of the anterior surface, as illustrated in **Figure 1**. A simple geometric formula resulted, which assumed the targeted corneal shape was a function only of the ablation profile. This can be thought of as the "shape-subtraction" paradigm, based on a geometric approach to tissue removal and secondary curvature change, where the final corneal shape is thought to be determined solely by how much tissue is 'subtracted' by the laser. Essentially, the cornea is a piece of plastic to be sculpted into the "ideal" surface shape.

The equations described by Munnerlyn et al still serve as a starting point for developing current ablation algorithms. For a myopic ablation, the pre-operative cornea is modeled as a sphere of greater curvature than the desired post-operative cornea, which is also modeled as a sphere. The apex of the desired post-operative cornea is displaced from the pre-operative cornea by the maximal ablation depth, which is determined by the ablation zone size. The intervening tissue is simply removed or "subtracted" to produce the final result. This is illustrated in **Figure 2** for a myopic, as well as a hyperopic profile which is similar in concept, but with a different planned ablation profile that produces increased post-operative curvature. This concept will be referred to as the "shape-subtraction" model of refractive surgery and treats the cornea as if it were a homogeneous structure, like a piece of plastic. The incomplete shape-subtraction paradigm permeates current thinking in refractive surgery, and forms the basis for both topography-guided and wavefront-guided procedures.

The Munnerlyn approach, combined with empirical experience, has been relatively successful in correcting spherical and cylindrical errors for the majority of the patient population to date. However, a substantial number of individuals do not achieve 20/20 vision (only 50 - 85% do achieve 20/20, depending on who is reporting), despite greater than 90% being satisfied. In addition, significant post-operative optical aberrations are produced using conventional algorithms, hence the move toward developing aberration-reducing ablation profiles.

After acceptance of PRK, laser in-situ keratomileusis (LASIK) was proposed as an alternative procedure. LASIK involves cutting a thin corneal flap of tissue, ablating within the stromal bed, and returning the flap. LASIK has become increasingly popular since it is associated with less pain and faster visual recovery. In addition, it was thought that the preservation of the corneal epithelium in LASIK would minimize regression. Ultimately, however, both procedures produce similar visual outcomes.

When these procedures failed to produce the expected refractive outcomes with consistency, it became clear that the model was incomplete. Growing appreciation for the complexity of the corneal response coupled with a rapidly growing body of clinical experience led to a more empirical approach to algorithm design. Current ablation algorithms are proprietary to the particular manufacturer, and it is not known how similar they are to the original formula. However, it is believed that the ablation profiles have been empirically altered based on experience with real clinical data.

Among a large array of possible input variables, the ablation algorithm is the most exquisitely controllable. Consequently, it is often the sole focus of refractive control in developing sophisticated corneal ablation routines based on topographic and/or wavefront analysis. However, the model of corneal response that contributed to unpredictability in noncustomized procedures still influences the refractive outcome and overall visual performance. Although empirically modified ablation algorithms have produced reasonable results in many patients, they are unlikely to provide the individual predictability that physicians desire and patients increasingly demand. Moreover, algorithms derived from these probabilistic models were optimized for the *mean population* response rather than that of the *individual,* and a certain degree of prediction error is inescapable. The current practical approach to excimer laser refractive surgery has become one of iteration between ablation recipe and surgical response, with most surgical decisions being made at the empirical level and confounded by an incomplete working knowledge of the comea's physical behavior during and after ablation. Individual surgeons use their own "fudge" factors, making systematic interpretation of results difficult Thus, the fundamental challenge to the future of custom corneal ablation is to develop deterministic models that can be successfully applied to the individual patient, rather than relying heavily on empirical modification. Important biomechanical determinants of corneal response to excimer laser ablation and a rationale for inclusion in customized corneal ablation models, presented herein, was previously unrecognized in ablative procedures.

The cornea has been modeled as a biomechanical structure for refractive procedures that do not rely on excimer laser ablation, such as radial keratotomy. (Roy et al., 1996; Hanna et al., 1992;Vito et al. 1989; Bryant et al., 2000; Pinsky and Datye; 1991a,b) In these existing numerical models for refractive surgery, the cornea is assumed to be a solid material and the effect of the surgically-induced structural change on corneal deformation, produced by an incisional or other procedure, is investigated. The only mechanical loading condition used in these simulations is the intraocular pressure applied to the posterior surface of the cornea. Living human cornea, however, is highly porous and filled with a biological fluid In fact, 80 % by weight is considered to be water. Also, the corneal endothelium serves as a biological "fluid-pump", maintaining a state of relative dehydration with a negative intra-stromal pressure (approximately 50 ~60 mmHg below the atmospheric pressure) called the "imbibition pressure". In addition, the microstructure of the corneal stroma consists of approximately 300 ~ 500 lamellar layers throughout its thickness. Each layer consists of collagen fibers supported by a mstrix consisting of "proteoglycans" and water. When living human cornea is incised or ablated, the long collagen fibers are severed in a series of layers. In addition to the loss of the tensile force in the remaining collagen segments, the water in the neighborhood of the damaged portion of the cornea can no longer maintain the same level of negative ("imbibition") pressure. This change in water pressure inside the damaged portion of the cornea plays a crucial role in corneal deformation. However, this factor has never been considered in any of the existing simulation methodologies for refractive surgeries.

The biomechanical response - as used herein, the term "biomechanical response," sometimes used interchangeably with. "biodynamic response," means a mechanical or physical response to a perturbation or other stimulus - has not been directly incorporated into current ablation algorithms. As a result, predictability of the visual result currently achievable, even in "customized" wavefront-guided or topography-guided procedures, has been inadequate. There are significant differences in the material properties of living human corneas based on age, sex, race, climate, years of contact lens wear, etc. Thus, there are significant differences in the response of various individuals to similar ablative profiles. The ability to predict the biomechanical response of the cornea to a photoablative procedure, and account for it in ablation algorithm design would significant enhance quality of vision produced. Knowledge of *individual* biomechanical properties, prediction of *individual* reponses, and *individual* adjustment of generalized ablation profiles would allow *individual* customization, rather than optimizing to the mean population response. Therefore, a biomechanical model with predictive capabilities would significantly enhance the overall success rates of these refractive surgeries.

### Summary of the Invention

The invention pertains most generally to improving visual quality through refractive surgery. An aspect of this general goal is to obtain the highest level of subjective satisfaction of visual quality as measured objectively. The gist of the invention resides in the predictive capability of the biodynamics of the eye. A wide variety of photoablative procedures can benefit from this predictive ability, in a number of ways. For example, LASIK or PRK treatments can be improved by taking pre-operative measurements and predicting the corneas's biodynamic response to the ablative treatment. In another embodiment of the invention, predictive use is made of the biodynamic response of the cornea due to laser or mechanical keratectomy, that is, creating a corneal flap characteristic of LASIK. Comparison of pre-flap and post-flap, (pre-ablation) data of the cornea such as corneal thickness, flap thickness, corneal topography and wavefront, for example, can provide predictive information applicable to modifying an ablation algorithm before the laser is engaged, either for a current operation or the development of a model. Modeling, by finite element analysis or other mathematical techniques, can also be used to predict post-operative outcomes based on a pre-operative (no flap cut or other surgical intervention) data for the cornea that is input for an accurate eye model that, in consideration of biodynamic response via the model, provides predictive information for optimizing the success of the refractive surgery and ultimately patient satisfaction. LASIK, PRK and other photoablative techniques benefit from these embodiments of the invention.

In accordance with the foregoing, an embodiment of the invention is directed to a method for performing refractive corneal surgery. The steps include obtaining a pre-operative (e.g.., pre-flap cut for LASIK) diagnostic measurement data of the eye, determining ablation specifications based upon the pre-operative diagnostic measurement data, perturbing the cornea of the eye such that there is a biodynamical response, obtaining a post-pertubation diagnostic measurement of the eye, determining an adjusted ablation specification based at least in part on a comparison of the pre-operative and post-pertubation data wherein these data are indicative of a biodynamical response of the eye, and ablating a portion of the cornea using the adjusted ablation specification. As will be evident to one skilled in the art, there will be a link between the measurement comparison and the biodynamic response. For example, the post-pertubation measurement may indicate a greater or lesser degree of central corneal flattening and greater or lesser degree of peripheral corneal steepening and thickening. These structural and physiological changes are manifestations of the magnitude of the biodynamic response of the cornea to the pertubation. The magnitude of the biodynamic response can then be used to adjust the ablation specifications, e.g., less central pulses and more peripheral pulses.

Another embodiment is directed to a method for establishing a photoablative specification by obtaining a pre-operative (e.g. pre-flap cut) diagnostic measurement data of the cornea, determining ablation specifications based upon the pre-operative diagnostic measurement data, perturbing the cornea of the eye such that there is a biodynamic response, obtaining a post-pertubation diagnostic measurement of the cornea, and determining an adjusted ablation specification based at least in part on a comparison of the pre-operative and post-pertubation data wherein these data are indicative of a biodynamical response of the eye.

Associated with the immediately foregoing embodiment is a method for correcting and, preferably, optimizing existing algorithms for photoablative ophthalmic surgery that includes taking data of a statistically significant number of corneas. Biomechanical response has not been directly factored into current ablation algorithms. With a proper biodynamic model, advance measurements of individual characteristics that are related to biodynamic response can be used to predict surgical outcomes. In previous biomechanical simulations of refractive surgeries, the cornea was treated as a solid material. In this invention, the highly porous nature of and the normal negative pressure within the cornea is taken into consideration. By considering the changes in the position-dependent negative ("imbibition") pressure due to the tissue removal thorough surgery inside the cornea, the central portion of corneal surface after surgery is shown to flatten. Therefore, through the inventive method, which includes the position-dependent negative "imbibition" pressure, the basic clinically, known results of LASIK or PRK can be correctly modeled. In addition, the clinically observed peripheral thickening after the LASIK or PRK is also demonstrated through the usage of simplified models.

In the existing simulations for refractive surgeries, only the mechanical loading external to the cornea such as the intraocular pressure is considered. In the invented simulations, the cornea is basically modeled as a fluid-filled porous material as opposed to a solid material in the existing simulations. Therefore, the mechanical loading inside the cornea such as the negative ("imbibition") pressure applied on the internal pore boundary is directly incorporated into the simulations. Because of this major difference between the two methods, the inventive methodologies can reproduce and predict actual clinical physical phenomenon.

Other advantages and benefits of this invention will be apparent from the following detailed description.

### Drawings

**Figure 1** is a schematic of the simple "shape-substraction" paradigm for correction of myopia.
**Figure 2** is a schematic diagram of shape-subtraction model of refractive surgery for myopic ablation (left) and hyperopic ablation (right).
**Figure 3** presents post-operative minus pre-operative *difference* maps: elevation (upper left), pachymetric (lower left), tangential curvature (upper right) and axial (lower right).
**Figure 4** is a tangential curvature difference map (post-op minus pre-op) after a LASIK procedure.
**Figure 5** is a pachymetry difference map (post-op minus pre-op) after a myopic LASIK procedure.
**Figure 6** has post-op minus pre-op difference maps from a LASIK correction.
**Figure 7** has error maps of actual minus predicted post-op topography for the same subject as Figure 6.
**Figures 8 and 9** present maps of: A) pre-operative elevation (left) and tangential curvature (right); B) post-operative elevation (left) and tangential curvature (right); and C) elevation error (left) and tangential curvature error (right).
**Figure 10** presents regression analysis of peripheral stromal thickness of the superior region (upper plot) and inferior region (lower plot) against curvature.
**Figure 11** is a conceptual model that predicts biomechanical central *flattening* as a direct consequence of severed corneal lamellae.
**Figure 12** is a schematic diagram of the forces described by A) the interlamellar cohesive strength and B) the interlamellar shear strength.
**Figure 13** illustrates the impact of biomechanics on LASIK nomograms for primary and secondary hyperopia.
**Figure 14** shows a sample ablation depth profile calculated at 6 months after PRK.
**Figure 15** is a comparison between planned ablation profiles and measured topographic elevation difference maps.
**Figure 16** has average difference maps between repeated measures of 20 eyes of 10 normal subjects.
**Figure 17** has average difference maps between 1 day post-op LASIK and pre-op state.

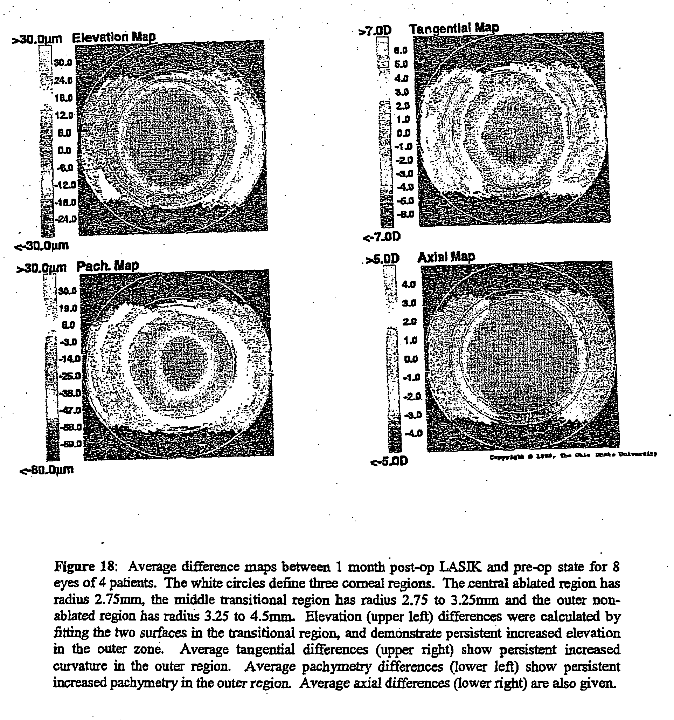

**Figure 18** has average difference maps between 1 month post-op LASIK and pre-op state.
**Figure 19** presents composite difference maps of peripheral increases in curvature (upper right), elevation (upper left), and thickness (lower left) for patients who underwent LASIK surgery and had Orbscan corneal topography pre-operatively and at 6 months post-operatively.
**Figure 20** presents optical image views of the central region (A) and the inferior (B) superior (C) regions of the cornea.
**Figure 21** graphs central curvature shifts vs. regional peripheral stromal thickness changes.
**Figure 22** shows a pre-operative (upper left) and (lower left) curvature topography taken while the patient is supine for a LASIK procedure.
**Figure 23** the curvature difference map between pre-op and post-flap for the patient in **Figure 22**, calculated and displayed using customized software. The curvature difference map between post-flap and post-ablation for the same patient.
**Figure 24** is a model for an intact cornea, presented as a portion of an axisymmetric layered spherical shell fixed at the ends.
**Figure 25** is a model for cut cornea after PRK or LASIK surgery.
**Figure 26** illustrates flattening of the model cornea after surgery based on the fluid-filled porous material assumption of cornea.
**Figure 27** illustrates bulging of the model cornea after surgery based on the solid material assumption of cornea
**Figure 28 and 29** compare the overall corneal deformed shapes for intact and cut corneal models, with two different assumptions.

### Detailed Description

In order to improve the percentage of patients who achieve post-operative visual acuity of 20/20 or better, as well as allow minimization of aberrations, it is important to critically examine our current conceptual model of laser refractive surgery. There are three assumptions inherent in the shape-subtraction model that are not supported by the data and yet currently drive algorithm development. These flawed assumptions are: 1) the only portion of the cornea that is changed is within the ablation zone; 2) what you cut is what you get; and 3) even if there are changes outside the ablation zone, they don't affect central vision.

Our comprehensive, integrated model of corneal response to laser refractive surgery takes into account three proposed components to the final corneal shape which determines vision: 1) ablation profile and laser parameters; 2) epithelial and stromal healing; and 3) biomechanical response to a change in structure. The third component, biomechanical response, has not been directly incorporated into existing ablation profiles in excimer laser refractive procedures. Therefore, this three pronged, systematic model, fully characterizing the corneal response represents an innovative approach to solving this complex problem. In addition, within the predictive model, an optimization approach is used to develop new ablation algorithms. Using optimization routines to produce the best possible outcome also represents a highly innovative approach to laser refractive surgery. This model and this optimization approach provide answers to the three assumptions or questions discussed above.

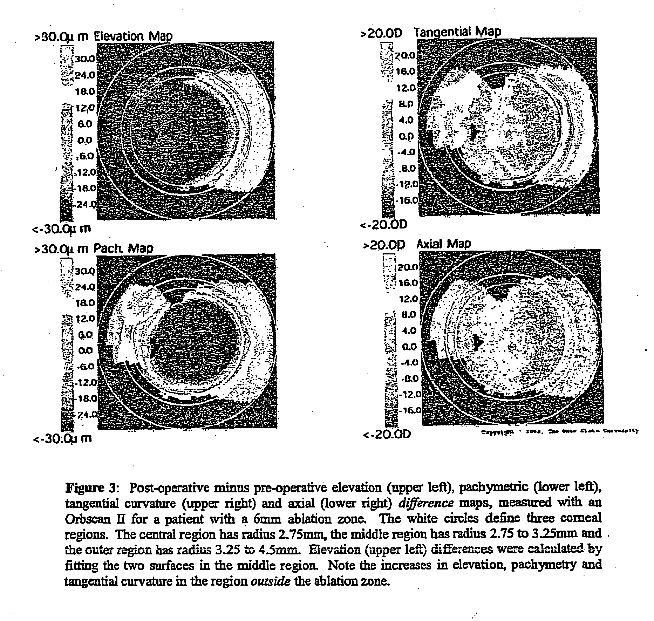

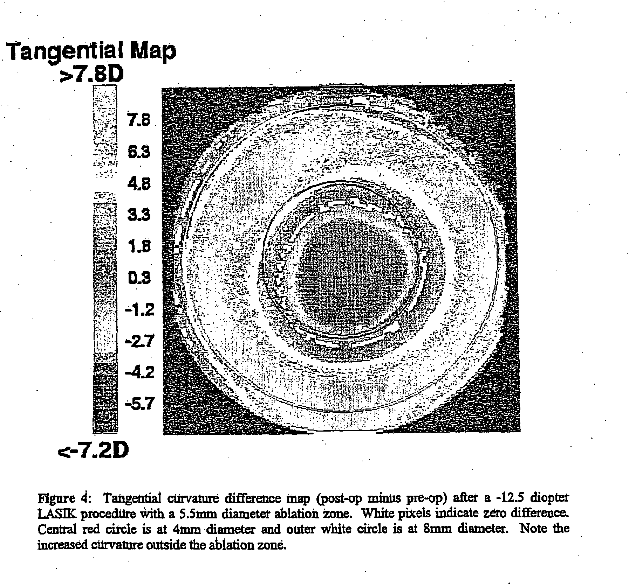

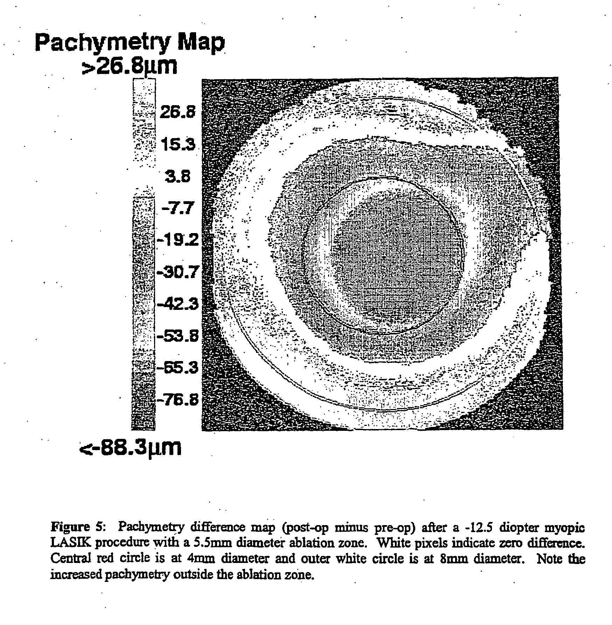

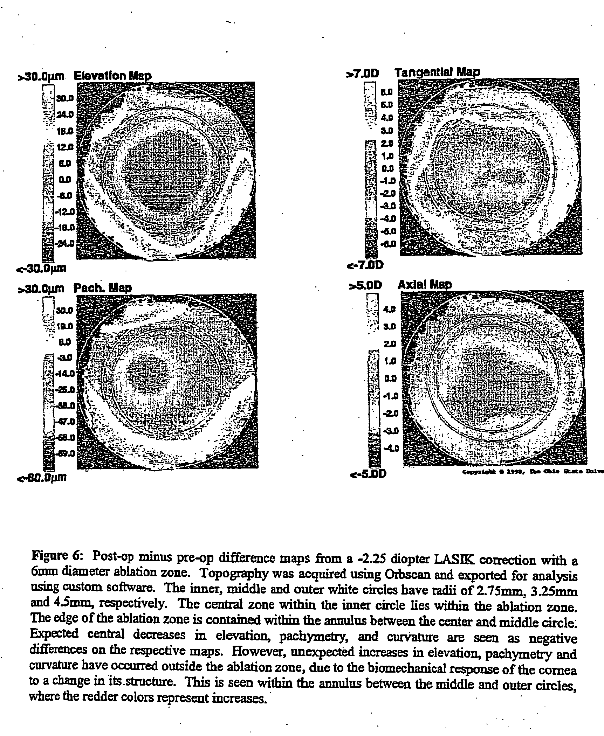

Assumption #1 can be invalidated by anyone with access to a set of post-operative topography from PRK or LASIK. Outside the ablation zone, curvature significantly increases with the appearance of the characteristic red ring (high dioptric value) surrounding the central flattened zone after a myopic procedure. (Shown in the *tangential* map, not the axial.) In addition, elevation and pachymetry also *increase* outside the ablation zone, as measured by Orbscan topography, and shown with an example in **Figure 3**. Similar examples from another preliminary study are shown in **Figure 4**, which is a tangential curvature difference map (post-op minus pre-op) after a -12.5 diopter LASIK procedure using a Technolas 217 (Munich, Germany) with a 5.5mm diameter ablation zone. The topography was acquired using an ORBSCAN I (Salt Lake City, Utah). The data were exported using the recorder function, and subsequently imported into custom software for analysis. Centrally, there is a decrease in curvature, as expected, indicated by the negative values and blue colors. The surrounding thin white area represents zero difference between the pre and post-op state. In the area outside the ablation zone, there is an unexpected increase in curvature which extends into the periphery, indicated by the positive values and red colors. Changes in corneal curvature clearly occur well beyond the ablation zone, challenging assumption #1. **Figure 5** is a pachymetry difference map from the same patient. Inside the ablation zone, the thickness has decreased, as expected. However, outside the ablation zone, the thickness has unexpectedly increased. In addition, regression analysis between the central and peripheral curvature changes showed a significant negative correlation (p<0.0053), indicating that greater central flattening produced greater peripheral steepening. The peripheral increase in curvature is a known consequence of laser refractive surgery, but has never been fully explained other than as a "knee" at the edge of the ablation profile. If this were an accurate description, the change would be confined near the edge of the ablation zone, and yet it extends well beyond that region. Additional data in the form of elevation and pachymetry maps, from the same patient population, also showed significant peripheral *increases* in both elevation and pachymetry *outside* the ablation zone, corresponding to the increase in curvature. These paradoxical changes in elevation and pachymetry have been anecdotally attributed to measurement device errors, without exploration of the responsible biomechanical mechanisms. **Figure 6** shows four difference maps from a sample patient in this population with a 6mm ablation zone, including elevation, tangential, axial, and pachymetry differences. The expected decreases in curvature, elevation and pachymetry centrally, within the ablation zone, are noted. However, the maps also show unexpected *increases* in elevation, pachymetry, and curvature *outside* the ablation zone. Therefore, the results of this study challenge the validity of the assumption that corneal curvature changes are confined to the ablation zone. The proposed mechanism for the paradoxical changes in elevation and pachymetry are the subj ect of this invention.

The results of the analysis of assumption #1 demonstrated that shape changes occur well beyond the ablation zone. Assumption #2 can be examined more directly by comparing ablation profiles to actual corneal surface changes. In a preliminary study, 10 LASIK patients without astigmatism who had symmetric ablation patterns, pre and post-operative topography was obtained using an Orbscan I. LASIK was performed using a Technolas 217 excimer laser. Ablation algorithms were approximated using Munnerlyn's formulas. The calculated ablation profile was subtracted from the pre-operative topography to generate a predicted post-operative topography. Actual post-operative topography was compared to predicted post-operative topography and error maps generated. RMS errors were calculated within the central 4mm diameter zone, outside the central zone, and over the entire map. Results averaged for all 10 subjects are given in **Table 1**, and demonstrate larger error outside the 4mm central zone than inside.

**TABLE 1: RMS Error between Predicted and Measured Topography after LASIK**

| | elevation mean (n=10) RMS error | curvature mean (n=10) RMS error |
|---|---|---|
| central 4mm diameter | 18 ± 14 microns | 4.92 ± 1.89 diopters |
| 4-9mm diameter zone | 23 ± 11 microns | 8.06 ± 1.76 diopters |
| overall | 22 ± 11 microns | 6.85 ± 1.50 diopters |

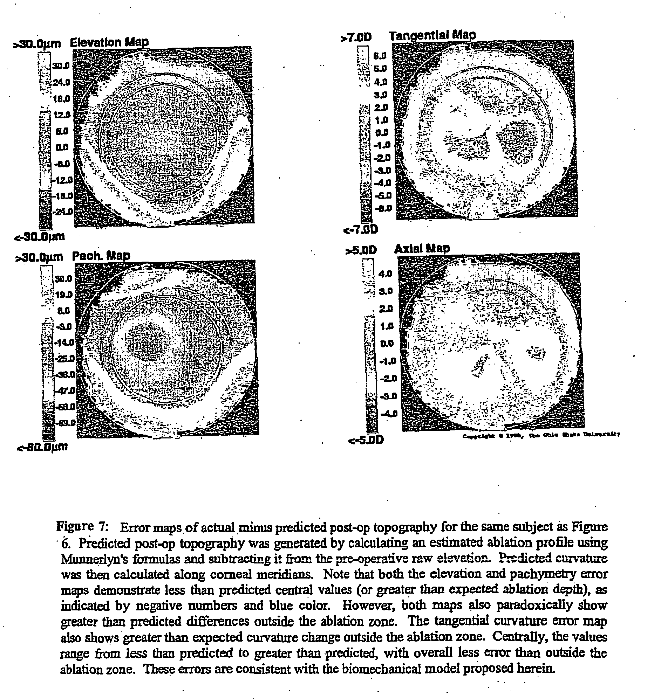

The sample error map in **Figure 7** shows a pattern of positive error peripherally in elevation, pachymetry, and curvature with negative error centrally in elevation, pachymetry and somewhat in curvature, although curvature is not consistently negative over the whole central region. These patterns of error cannot simply be attributed to using an estimated rather than known ablation profile, since they are both nonrandom and nonlinear. Ideally, the actual ablation algorithms should be used in a study of this type, rather than Munnerlyn's formulas, but they were not available due to their proprietary nature. However, despite using estimated ablation profiles, the evidence presented challenges the validity of assumption #2. These patterns of error are not consistent with a shape-subtraction model of ablation, but they are consistent with the biomechanical model to be proposed below.

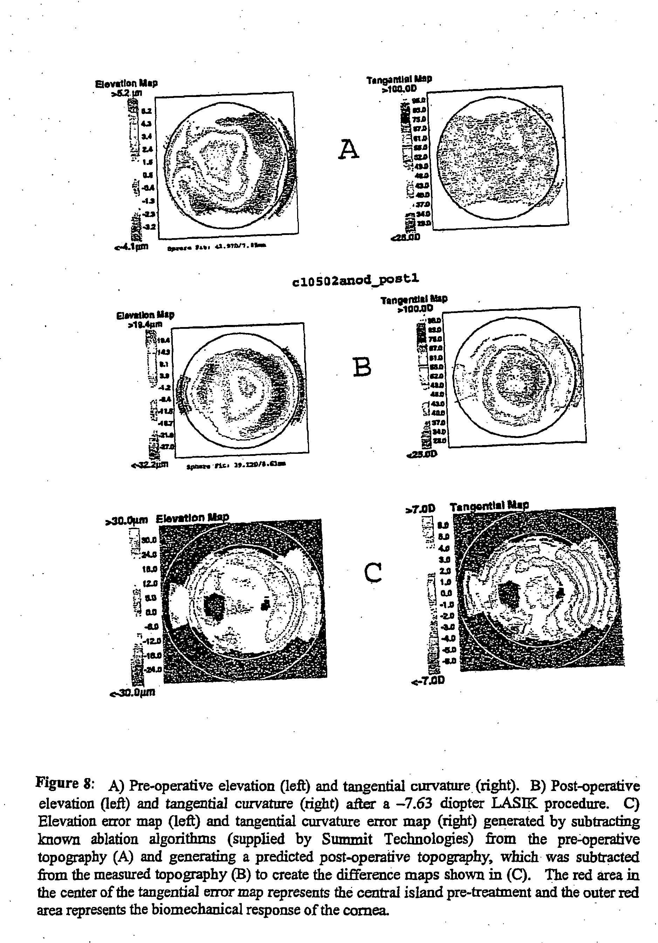

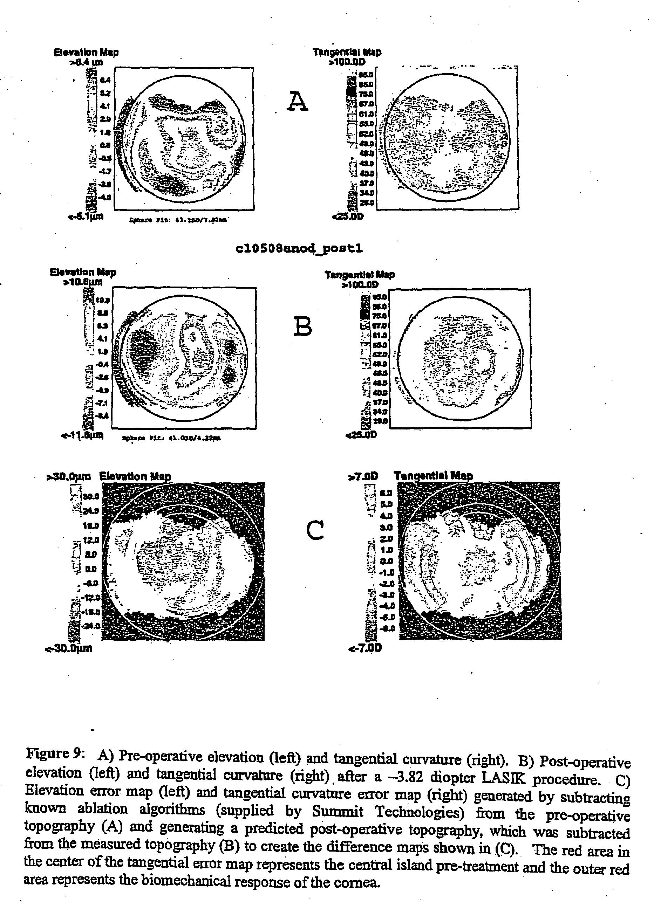

Assumption #2 can be further invalidated by examining the predicted topographic result with a known ablation profile, and comparing that to the measured result. In another preliminary study, Summit Technologies agreed to supply their proprietary ablation algorithms for several LASIK patients being treated with a Summit Apex Plus using a Krumeich-Barraquer microkeratome. The actual ablation algorithm was subtracted from the pre-operative topography (measured with an Orbscan II) to generate a "predicted" post-operative topography. The predicted topography was subtracted from the measured post-operative topography to generate "error" maps, which are given in **Figures 8 and 9** for two patients, along with the measured results. Several important features should be noted. First, the red area in the center of the tangential curvature error maps correspond to the central island treatment. This treatment involves delivery of extra pulses in the center of the cornea, which *would* cause excessive flattening *IF* they were not compensating for the central island phenomenon - the genesis of which is not yet understood. Therefore, the curvature error maps appears to have a "central island" even though the post-operative topographies are smooth. Second, the red areas *outside* the ablation zone on the tangential curvature error maps correspond to the unexpected increases in curvature, which are not predicted by the shape-subtraction model, and are predicted via the biomechanical model presented herein.

Finally, how can documented changes outside the ablation zone affect central curvature (assumption #3)? The answer to this question lies in the biomechanics of the corneal response to laser refractive surgery, which is unaccounted for in current ablation algorithms. It has been known since the inception of refractive surgery that altering the corneal structure will alter the shape of the entire cornea, whether using an incisional or thermal mechanism. Fundamentally, if the cornea were a piece of plastic, radial keratotomy would not have worked! Yet, with the development of laser refractive surgery, the structural link between the central and peripheral cornea was ignored. The cornea was thought of quite simply as a homogeneous structure to be "sculpted" into a new shape. However, this conceptualization cannot account for all of the corneal shape changes that occur after an ablative procedure. An important component of the proposed biomechanical model of corneal response to laser refractive surgery, to be described in the next section, is peripheral stromal thickening or an *increase* in corneal elevation *outside* the ablation zone. Evidence that this occurs was presented in the analysis of assumption #1. However, the question remains as to the linking of the central and peripheral corneal events. Is the peripheral increase in corneal elevation statistically correlated to central curvature changes, or are they independent, unrelated occurrences? Regression analysis between central curvature change and peripheral elevation change from the 30 subjects who underwent LASIK demonstrated a significant positive correlation (R² = 0.56, p < 0.0001), indicating that the greater the increase in elevation outside the ablation zone, the greater the curvature change (flattening) centrally. This provides evidence that the peripheral response of the cornea is linked to the central response, although it does not account for all the variance.

The following case study further illustrates how central curvature changes can actually track peripheral thickness changes after ablation. The patient had 6mm diameter PRK (VISX Star) for a refractive error of -3.75 + 0.75 x 90, with Orbscan I topography pre and post-operatively. Both superior and inferior corneal thicknesses were calculated by averaging a 1mm diameter region in the pachymetry map along the vertical meridian, at the edges of a 7.5mm diameter circle centered on the ablation zone. Central curvature was calculated by averaging the curvature in the 3mm diameter central region of the tangential map. The values as a function of time relative to surgery are given in Table 2.

**TABLE 2: Case Study of Curvature and Thickness Changes after 6 mm Diameter PRK**

| | Superior Thickness (microns) | Inferior Thickness (microns) | Central Thickness (microns) | Central Curvature |
|---|---|---|---|---|
| preop | 715 | 682 | 605 | 41.55 diopters |
| 30 minutes | 770 | 729 | 710 | 36.20 diopters |
| day 4 | 727 | 691 | 589 | 39.39 diopters |
| 3 weeks | 744 | 701 | 568 | 38.81 diopters |
| 3 months | 722 | 690 | 570 | 39.32 diopters |

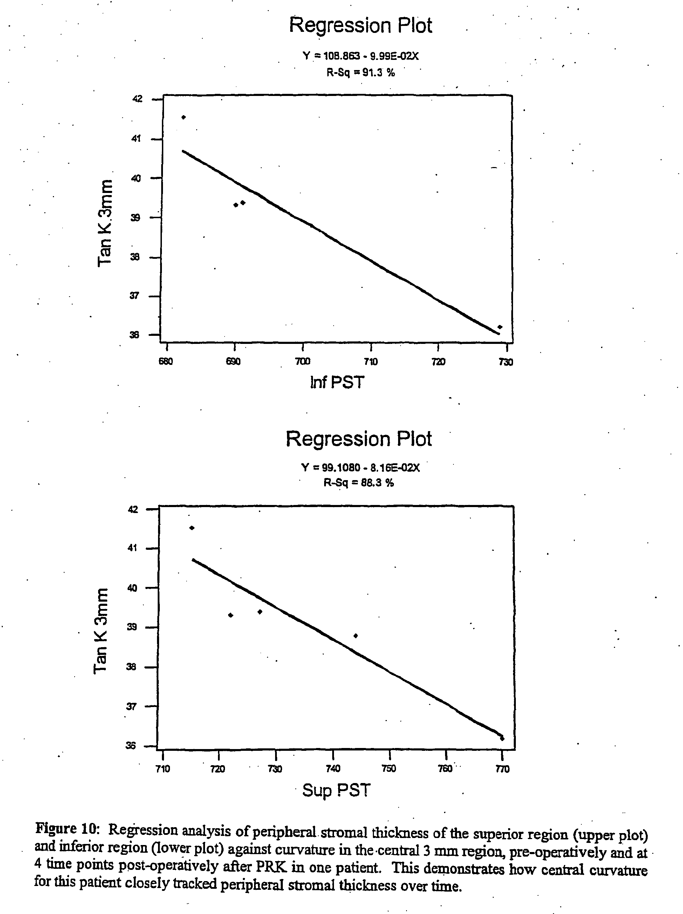

Note that both central curvature and peripheral thickness demonstrate post-operative fluctuation in opposite directions. Regression analysis of central curvature vs peripheral thickness was performed, and the plots are given in **Figure 10**. Central curvature has a strong negative correlation with peripheral thickness, both inferior and superior, meaning the greater the peripheral thickness, the flatter the central curvature. This provides further support that the central and peripheral changes are linked and assumption #3 is not valid.

Evidence has been presented challenging the inherent assumptions of the shape-subtraction model of refractive surgery. Clinical data indicate that substantial changes occur outside the ablation zone that are structurally linked to central curvature, and may affect central vision. The proposed mechanism for the measured increases in elevation, thickness, and curvature which occur outside the ablation zone will be presented below.

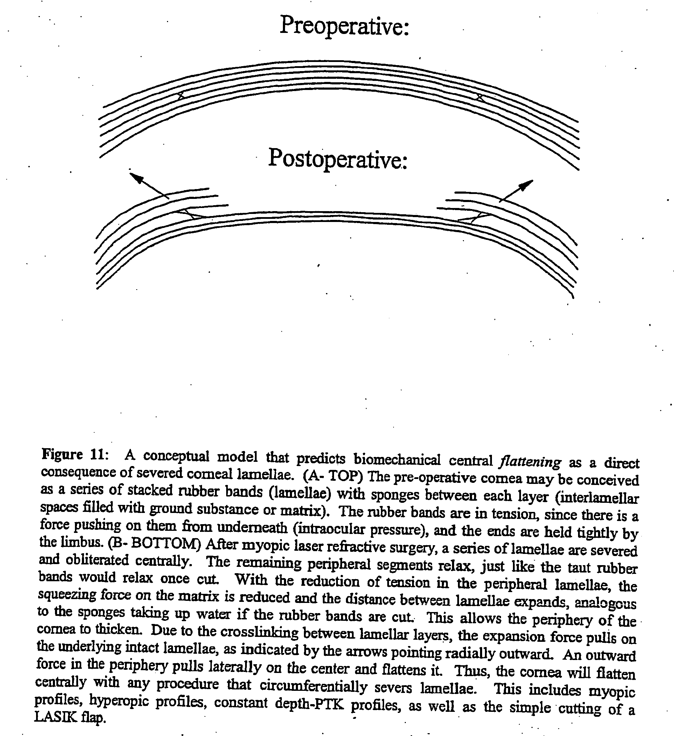

A conceptual model is presented in **FIGURE 11** that predicts biomechanical central *flattening* as a direct consequence of severed corneal lamellae. Rather than a piece of plastic, the cornea may be conceived as a series of stacked rubber bands (lamellae) with sponges between each layer (interlamellar spaces filled with ground substance or matrix). The rubber bands are in tension, since there is a force pushing on them from underneath (intraocular pressure), and the ends are held tightly by the limbus. The amount of water that each sponge can hold is determined by how tautly the rubber bands are pulled. The more they are pulled, the greater the tension each carries, the more water is squeezed out of the interleaving sponges, and the smaller the interlamellar spacing. This is analogous to the pre-operative condition in **Figure 11A.** After myopic laser refractive surgery, a series of lamellae are severed centrally and removed, as shown in **Figure 11B**. The remaining peripheral segments relax, just like the taut rubber bands would relax once cut. With the reduction of tension in the lamellae, the squeezing force on the matrix is reduced and the distance between lamellae expands (negative intra-stromal fluid pressure), analogous to the sponges taking up water if the rubber bands are cut. This allows the periphery of the cornea to thicken. Due to the crosslinking between lamellar layers, the expansion force pulls on the underlying intact lamellae, as indicated by the arrows pointing radially outward. An outward force in the periphery pulls laterally on the center and flattens it. Thus, the cornea will flatten centrally with any procedure that circumferentially severs lamellae. The biomechanical flattening enhances a myopic procedure, works against a hyperopic procedure, and will cause flattening in a "non-refractive" PTK. This includes myopic profiles, hyperopic profiles, constant depth-PTK profiles, as well as the simple cutting of a LASIK flap.

In every LASIK procedure, a flap is cut with a microkeratome to a thickness of approximately 160 microns. Biomechanically, this is very close to a 160 micron depth ablation, and according to the proposed model, should induce corneal flattening. The amount of flattening produced should be indicative of the biomechanical response of the cornea to refractive surgery. Topography of this epithelial surface of the cornea, before and just after cutting the flap, permits alteration of ablation algorithms in real time to account for individual biomechanics, and improves surgical outcomes.

### A Biomechanical Model of Keratectomy-Induced Curvature Change

In recent decades, a great deal of effort has been devoted to characterizing the cornea as a biomechanical entity. This work has included *in vitro* measurement of key material properties such as the modulus of elasticity and shear modulus, and numerous computational models have been generated to approximate the structural response to simulated incisional keratectomy. While these efforts have undoubtedly advanced the basic understanding of corneal behavior under certain specific conditions, less has been done relative to modeling the structural changes induced by laser keratectomy, and the predictive value of existing numerical models is limited by their implicit simplifications (e.g. modeling the cornea as a solid substance).

### Mechanical Model of Keratectomy-Induced Corneal Flattening

The basis for the proposed biomechanical theory of corneal response lies in the lamellar structure of the corneal stoma, which is altered in PTK, PRK, and LASIK. It is known the stroma dominates the mechanical response of the cornea to injury. The stromal lamellae, by virtue of their extension across the corneal width and continuity with the corneo-scleral limbus, bear a tensile load arising from intraocular pressure and extraocular muscle tension. The propensity of the stroma to imbibe fluid and swell, which is attributed to the hydrophilic macromolecules of the extracellular matrix, is resisted by the corneal limiting layers, the metabolic activity of the corneal endothelium, and the compressive effects of lamellar tension. The thickness of the stroma is lineraly related to the hydration and thus is an accessible indicator of changes in corneal mechanical equilibrium. It is proposed that when tension-bearing lamellae are disrupted by central keratectomy, their peripheral segments relax, causing local decompression of the extracellular matrix, and a compensatory influx of stromal fluid adding to increases in interlamellar spacing and peripheral thickness. Increases in thickness are limited ultimately by interlamellar crosslinks, which are preferentially distributed in the anterior one-third and periphery of the stroma, as depicted schematically in **Figure 11**, and are postulated to contribute to regional differences in lamellar shearing strength and interlamellar cohesive strength. A pivotal aspect of the model is the prospect that interlamellar stress generated in the expanding stromal periphery is communicated through this lattice of crosslinks to lamellae comprising the postoperative optical surface. Arrows in **Figure 11** indicate expansion of the peripheral stromal matrix secondary to ablation-induced lamellar relaxation. Peripheral thickening is proposed to exert an anterolateral tension at the ablation margin and stimulate central flattening. Coupling of peripheral and central tensile loads is mediated by interlamellar cohesive forces (''x'') preferentially distributed in the anterior-peripheral stroma (shaded). Components of force in the direction of peripheral expansion result in central corneal flattening and peripheral steepening.

During PRK, PTK and LASIK, central ablation causes an immediate circumferential severing of corneal lamellae under tension, with a subsequent relaxation of the corresponding peripheral lamellar segments. This causes a peripheral decompression of the extracellular matrix and an increase in stromal thickness outside of the ablation zone. It is further hypothesized that this response has important effects on central curvature due partly to the presence of interlamellar crosslinking, which predominates in the anterior one-third and periphery of the stroma and has been associated with significant lamellar shearing strength and interlamellar cohesive strength. Interlamellar stress is generated in the expanding stromal periphery and may be transferred through this network of crosslinks to the underlying lamellae, whose central portions comprise the postoperative anterior surface (Figure 11). The outward expansive force in the periphery causes the central cornea to flatten, independent of the ablation profile. Thus, even in the absence of a myopic (centrally weighted) ablation pattern, biomechanical changes in the cornea may produce an acute postoperative flattening of the central cornea and a refractive shift toward hyperopia. This response is perhaps most clearly demonstrated by the clinical phenomenon of unintended hyperopic shift in PTK, but its contribution to refractive variability in PRK and LASIK is also important, since biomechanical central flattening will enhance a procedure to treat myopia and oppose a procedure to treat hyperopia. An additional feature of the model -- an acute depth-dependent response - will also be discussed.

Of the five anatomic layers of the cornea, (the epithelium, Bowman's layer, the stroma, Descemet's layer and the endothelium), only Bowman's zone and the stroma contain collagen fibrils. These layers are thus presumed to provide the majority of the corneas' tensile strength. Although tension in the superficial epithelial cells has been cited as a potential mechanism for maintaining a smooth optical surface in the presence of underlying stromal surface undulations, removal of the epithelium causes little or no change in the anterior corneal curvature, and the epithelium is generally attributed a minimal role in corneal tensile strength.

The mechanics of Descemet's layer - the 7-um-thick hypertrophied basil lamina of the underlying endothelium - were studied in intact human and rabbit globes, and compared to findings in stroma from the same species. Stress-strain investigations revealed that in the *in situ* human cornea, the stromal stress-strain curve is quite steep, corresponding to a high Young's modulus, while Descemet's layer is highly extensible. Superimposing the stress-strain curves for the two layers demonstrated that Descemet's layer is essentially unstrained over a large range of intraocular pressures for which the stroma is simultaneously fully strained. This leads to the conclusion that, like the epithelium, Decemet's layer probably does not bear a significant proportion of the corneal tension over a physiologic range of pressures. Marked differences in the stress-strain relationships of rabbit and human stroma highlight the need for critical interpretation when using the rabbit as a model for corneal mechanical behavior in humans.

Structurally, Bowman's zone is little more than an acellular extension of the anterior stroma. Relative to the stroma, the individual collagen fibrils are two-thirds smaller (20 to 25 nm in diameter) and more randomly oriented throughout the 8 to' 12-um-thick sheet. However, it has long been believed that Bowman's zone contributes a structural rigidity to the cornea that is distinct from that provided by the stroma. But recent studies, using a linear extensiometer to examine a number of mechanical parameters in deepithelialized corneal strips obtained from fresh human cadaver eyes have indicated that removal of Bowman's layer did not significantly alter the constitutive mechanical properties of the cornea.

Finally, the stroma, which makes up about 90% of the total corneal thickness, is the layer thought to most significantly influence the mechanical response of the cornea to injury. The stroma is approximately 78% water by weight, 15% collagen, and 7% other proteins, proteoglycans, and salts. Three hundred to five hundred lamellae -- flattened bundles of parallel collagen fibrils -- run from limbus to limbus without interruption. In the posterior two-thirds of the stroma, the lamellae are successively stacked parallel to the corneal surface such that each lamellae has an angular offset from its anterior and posterior neighbors. Anteriorly, the lamellae are more randomly oriented, often obliquely to the corneal surface, are more branched, and are significantly interwoven. These regional differences correlate well with observations that shearing of the lamellae is generally difficult in the human stroma, but particularly so anteriorly. Collagen interweaving is also more extensive in the corneal periphery than in its center.

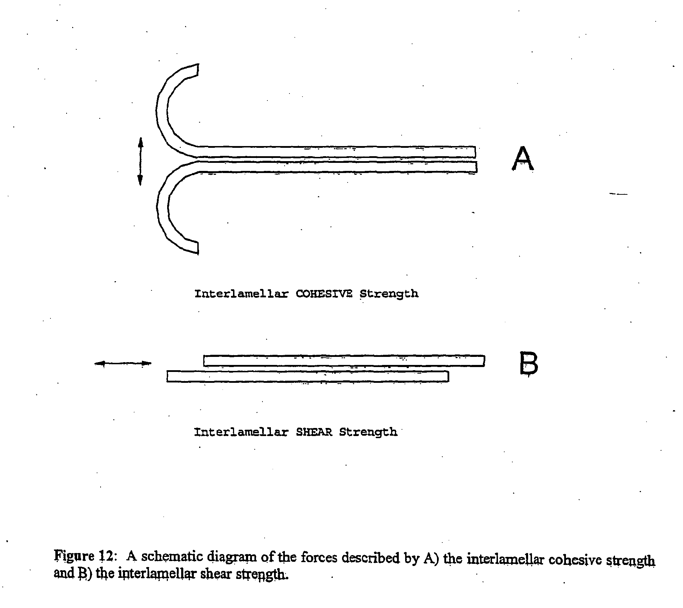

It is important to distinguish between cohesive strength and interlamellar shear strength as the two terms are frequently used interchangeably. *Cohesive strength* has been measured as the force required to separate a stromal sample along a cleavage plane parallel to the lamellar axes by pulling in a direction *perpendicular* to the cleavage plane, like peeling a banana (as shown in **Figure 12A**). Thus, the cohesive strength is a measure of the interlamellar resistance to separation in the transverse direction and is expressed as a function of distance from the corneal center. Alternatively, the *shear strength* manifests as a resistance to shearing or sliding of one lamellae over another in the plane *parallel* to the lamellar axes (the longitudinal direction); as such, it is an integrated function of the connective forces across the entire lamellar interface and is therefore conceivably larger in magnitude (**Figure 12B**). Both forces are likely to contribute to the peripheral-to-central transfer of stress in the proposed biomechanical model of corneal response to laser ablation.

Though the interlamellar cohesive strengths of the temporal and nasal peripheries of the horizontal meridian were equivalent in the study referred to above, cursory studies of the vertical meridian revealed large and consistent differences between the strengths of the superior and inferior regions. These differences were confirmed in a study which revealed a mean central strength of 0.165 ± 0.0088 N/min and mean peripheral strengths at 4-mm from the central cornea of 0.185 ± 0.0088 N/mm and 0.234 ± 0.0137 N/mm (p < .01) for the inferior and superior regions, respectively. Again, interlamellar cohesive strength in the human corneal stroma was shown to be greater in the periphery than in the center.

These studies provided anatomical and mechanical evidence of an interlamellar cohesion strength at 50% depth that is greater peripherally than centrally and greater superiorly than inferiorly. The predominance of interlamellar binding in the anterior one-third of the stroma indicates that the magnitudes of these forces are even greater in that region. Whatever bearing these interlamellar relationships have on the shape of the structurally intact cornea, their importance is likely to increase considerably in the ablated cornea when a redistribution of tensile loading induces non-physiological stresses between cut and uncut lamellae as illustrated in **Figure 11**. Furthermore, the asymmetric distribution of cohesive strengths can be an important source of induced corneal astigmatism in keratorefractive procedures.

The lamellar organization of the stroma and the capacity of this collagenous network to bear tension is a natural starting point for biomechanical models of curvature change. A relationship that is generally neglected in this context, however, is the relationship between the interfibrillary constituents of the stroma and water, the major component of the stroma. The collagen fibers are enmeshed in a ground matrix of glycosaminoglycans (GAG) such as keratan sulfate and chondroitin sulfates of varying degrees of sulfation. Both substances, but particularly chondroitin sulfate, are markedly hydrophilic and contribute to a negative intrastromal fluid pressure under which the entire stroma is heavily compressed. Intraocular pressure further compresses the stroma through its direct effect at the posterior surface and by its contribution to lamellar tension. The intrastromal pressure, often called the "swelling" pressure because of its tendency to draw water into the stromal ground substance, has been measured as -50 to -60 mmHg through a variety of *in vitro* and *in vivo* techniques.

In the normal physiologic state, this swelling tendency is resisted and relative dehydration is maintained by a combination of lamellar tension, anterior evaporation of the tear film, low permeability of the epithelial and endothelial layers to water, and active endothelial transport of bicarbonate. During the act of central ablation, however, a number of lamellae proportional to the depth of ablation are obliterated centrally and tension is lost in their unablated peripheral segments. The resulting loss of compression introduces a hydrostatic disequilibrium, and the peripheral stroma thickens as it takes up fluid. While tissue expansion is likely to occur within the ground substance rather than in the fibrils themselves, the source of the fluid has not been discerned. *In situ,* the cornea may imbibe additional water through the limbus as a result of the pressure difference between the perilimbal capillaries and the stromal interstitium. As one would expect, the swelling pressure diminishes as stromal hydration and thickness increase and a new steady-state is established.

As stated earlier, the essential link between peripheral stromal thickening and central flattening is the existence of a mechanical relationship between disrupted and intact lamellae. Contrarily, if lamellae are assumed instead to be structurally and mechanically independent of neighboring lamellae―that is, arranged in layers without any interconnections―then the severed peripheral segments are unable to bear or transfer any tension. This is, in fact, a simplifying assumption incorporated into most numerical models of refractive surgery. Within this scenario, the tensile load previously borne by the full complement of lamellae is shifted to the remaining posterior fibers, which now strain (stretch) slightly under the concentrated stress. If the limbal circumference is assumed to be fixed, the stretch may not be accomplished by an increase in corneal diameter and must therefore occur as central corneal bulging and anterior *steepening.* However, if the proposed peripheral response is considered, this occurs as a peripheral thickening and peripheral steepening with coincident central *flattening.* Although the latter scenario aptly describes the characteristic peripheral "knee" and central flat zone of PTK, PRK and LASIK, finite element analyses of uniform thickness profiles have not incorporated the peripheral response and have predicted a corneal configuration opposite that produced clinically. In short, consideration of the proposed peripheral stromal response and its mechanical relationship to the central cornea, as well as the negative intrastromal pressure, is critical not only for correctly predicting the *magnitude* of refractive correction, but in some cases, the *direction.*

### Peripheral Thickening Affects Central Curvature

One of the important implications of this work is the notion that shape changes measured *outside* of the ablation zone can have a significant impact on curvature changes *within* the ablation zone. The ablation zone analysis demonstrated in a controlled fashion that central curvature change in refractive surgery is not solely a product of the ablation pattern. When peripheral thickness and ablation zone bias were both included in a regression model, over 83% of the variance in curvature response accounted for by the model was explained by peripheral thickening. Although the impact of ablation pattern on acute curvature change is likely to become increasingly influential when larger dioptric corrections are attempted, the associated increases in ablation depth would also be expected to further exacerbate the biomechanical response. Consequently, the utility of a pure shape-subtraction model is probably limited to cases involving only superficial ablation (i.e., correction of low refractive error or astigmatism in PRK, and removal of subepithelial scars in PTK). Finally, the argument for the role of lamellar interconnections playing an important role was bolstered by the correlations between central flattening and thickness changes in individual peripheral subregions. These correlations were strongest in the superior periphery, weakest in the inferior periphery, and strongest overall in the far superior periphery―a pattern that consistently reflects the strength distribution of interlamellar cohesive forces reviewed above.

### Hyperopic vs Myopic Corrections

The proposed biomechanical model predicts additional flattening over and above whatever ablation profile is programmed, whether myopic with an intent to flatten, hyperopic with an attempt to steepen, or nonrefractive PTK. This would theoretically make hyperopia a more difficult procedure, since the biomechanical flattening would be in opposition to the ablation profile, thus requiring a deeper ablation to offset the biomechanical response. This prediction was verified in the first 8 patients treated with the Autonomous Custom Cornea, wavefront-guided ablation procedure. Of the 8. patients treated, 5 were myopic and 3 were hyperopic. Only the wavefront data were used to program the laser. Neither refraction, nor empirical experience were used. All 5 of the myopic patients were overcorrected (additional biomechanical flattening in combination with programmed ablative flattening) and all 3 hyperopic patients were undercorrected (biomechanical flattening in opposition to programmed ablative steepening).

In a comparison of primary vs secondary hyperopic treatments (overcorrected post-myopic procedures), primary hyperopic procedures require a treatment of up to 35% greater than the spherical equivalent, meaning greater than expected depth must be ablated to achieve the desired correction. This is consistent with the proposed model predicting biomechanical flattening, independent of the ablation profile. Greater depth must be ablated with a primary hyperopic treatment to overcome the biomechanical flattening which creates a surface shape effect opposing that of the ablation profile.

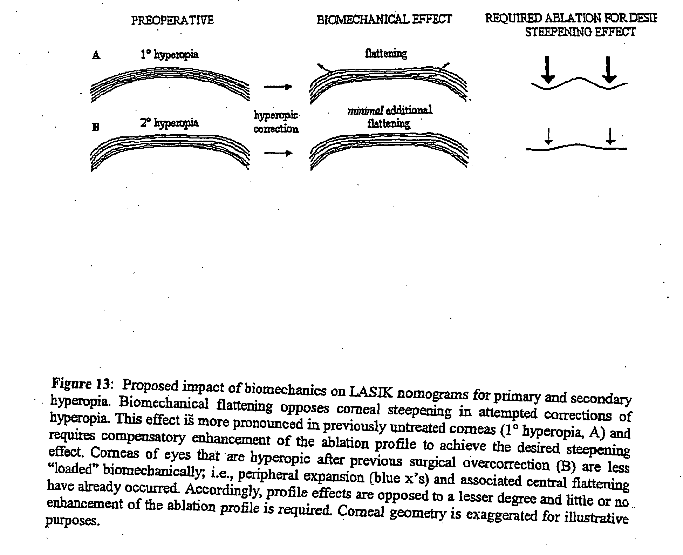

The secondary hyperopic group required substantially less depth of ablation to achieve the same level of correction as the primary hyperopia group. This is also completely consistent with the proposed biomechanical model. The secondary post-LASIK hyperopic group already had an altered corneal structure with an associated biomechanical response from the first refractive procedure. In other words, there was less biomechanical flattening to overcome in the second procedure, thus requiring less depth of ablation for the same correction, since the biomechanical effect had already occurred in conjunction with the first procedure, as illustrated in **Figure 13**. Third, the secondary hyperopes had a more stable longer term refraction, with the primary hyperopes exhibiting slight regression over the 6 month post-operative follow-up period. One possible explanation for this difference lies in the long-term healing related to the biomechanical effect, which would be exaggerated in the primary hyperopia group, as opposed to the secondary hyperopia group where the biomechanical effects had already occurred and were thus reduced.

### Peripheral Thickening After Laser Refractive Surgery In Vivo

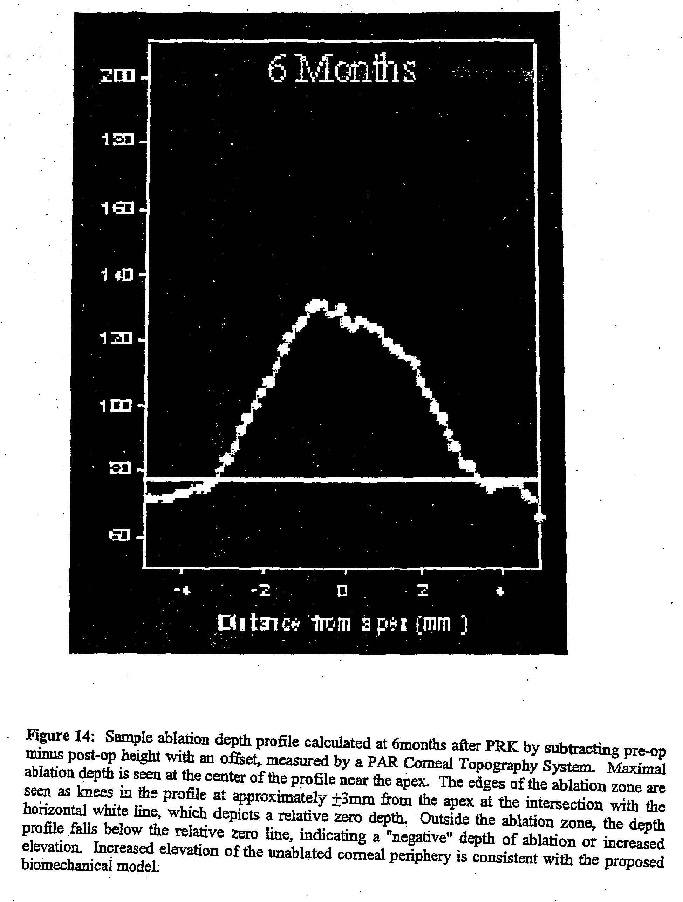

Indirect evidence of peripheral thickening after laser refractive surgery has been reported, which is consistent with the proposed model of biomechanical response. Munger, et. al. presented clinical results using a VISX Star system on a total of 25 eyes from 25 patients with low astigmatism, treated with myopic PRK. (Munger R, Jackson W B, Mintsioulis G, Ablation Profile and Epithelial Regrowth after Myopic PRK with the VISX Star. *American Society of Cataract and Refractive Surgery Annual Meeting,* 1999) Corneal maps were acquired with PAR Corneal Topography System (CTS) at 2 hours before surgery; 2-3 minutes after surgery (Sx); on the day the bandage contact lens was removed, and 1 week, 3 months, and 6 months post-operatively. Ablation depth was defined as preop - post-op, and epithelial depth was defined as Sx - post-op, recognizing that edema may have been a factor in the immediate post-op map. Munger reported epithelial thickening at the ablation edge. However, an increase in the calculated epithelial measurement outside the ablation zone may be due to *stromal* thickening rather than epithelial, since the PAR CTS measures surface elevation and cannot distinguish between epithelial and stromal thickening. In addition, more convincing evidence lay in the ablation depth measurements as a function of distance from the apex at 6 months, an example of which is shown in **Figure 14**. Ablation depth is a more robust measurement than the much smaller epithelial thickness, and by 6 months the cornea should be relatively stable. Note that outside the ablation zone, there is negative ablation depth, or increased elevation compared to the pre-operative state. This provides indirect evidence of peripheral thickening, an important feature of the proposed biomechanical model, with a different laser/topographer combination.

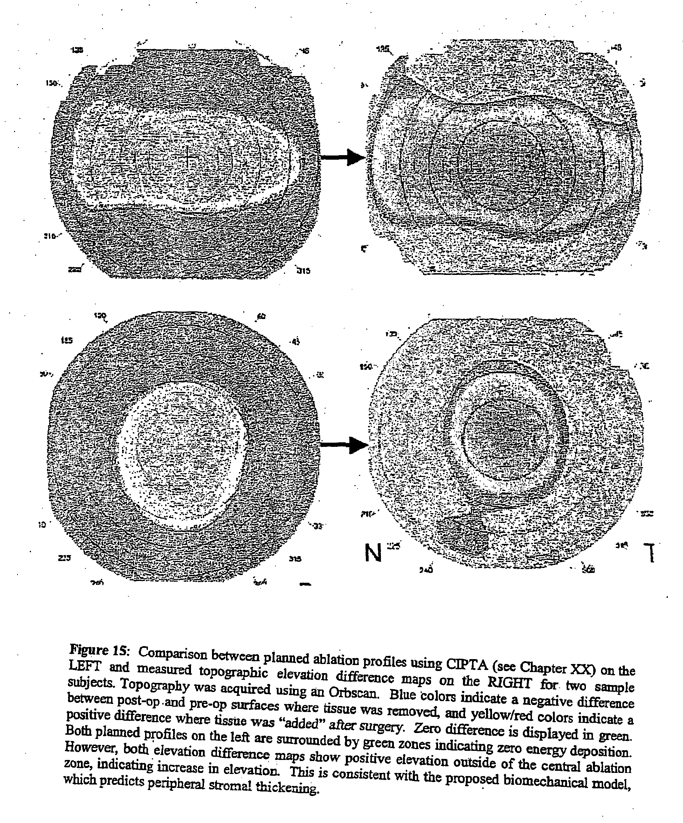

Sborgia, et al, has reported results of a corneal topography guided system called Corneal Interactive Programmed Topographic Ablation (CIPTA). (Sborgia C, Alessio G, Boscia F, Vetrugno M. Corneal Interactived Programmed Topographic Ablation: Preliminary Results. *American Society of Cataract and Refractive Surgery Annual Meeting* 1999) A total of 28 eyes of 28 patients with irregular astigmatism were treated with a Laserscan 2000 and had pre and post-operative topography with an Orbscan. Two examples of the planned ablation profile and actual elevation difference maps are given in **Figure 15**. In both subjects, the left image is the planned ablation pattern, and the right image is the actual elevation difference map. Note for both cases the large green area outside the ablation zone in the maps on the left, where the cornea will not be ablated. However, the elevation difference maps on the right demonstrate not only a decrease in central elevation (shown in blue) which matches the planned profile, but are also accompanied by an unexpected *increase* in elevation outside the ablation zone (shown in red and yellow), again as predicted by the proposed biomechanical model.

### Characterization of the Biomechanical Response

In a study to characterize the biomechanical corneal response to LASIK, and separate the resulting refractive effect from that produced by the ablation profile, 8 eyes of 4 subjects have been enrolled and analyzed. LASIK is performed with a Summit Apex Plus using a Krumeich-Barraquer microkeratome to make the flap. Corneal measurements are acquired pre and post-operatively with optical coherence tomography and the following corneal topographers for validation of effect: EyeSys, Humphrey Atlas, Keratron, Orbscan II, PAR, Technomed C-Scan, and TMS-1. Average pre-operative refractive error of the small preliminary group is -6.875±2.03 diopters sphere + 0.8125±0.51 diopters cylinder. Only the Orbscan corneal topography data will be presented here. A comparison control group of 20 eyes of 10 subjects had repeated Orbscan I topography acquired at intervals of from 1-2 days to establish baseline variations. The anterior tangential, anterior elevation and pachymetry data were exported to a customized topography tool for analysis and the cornea was divided into three regions: central 2.75 mm radius (5.5mm diameter), transition zone from a radius of 2.75 - 3.25mm (5.5-6.5 mm diameter), and outside the ablation zone from a radius of 3.25 - 4.5mm (6.5-9.0mm diameter). The pre-operative topography was subtracted from the post-operative topography for the surgical patients, and the repeated measurements were subtracted for the normal subjects. For the elevation maps, the two surfaces were fit within the 0.5mm transition zone. For all maps, average regional differences were calculated over the normal and surgical populations, and statistical analysis was performed using the ANOVA ("Analysis, of Variance") procedure in the software package, (Statistical Analysis System, Cary, NC)

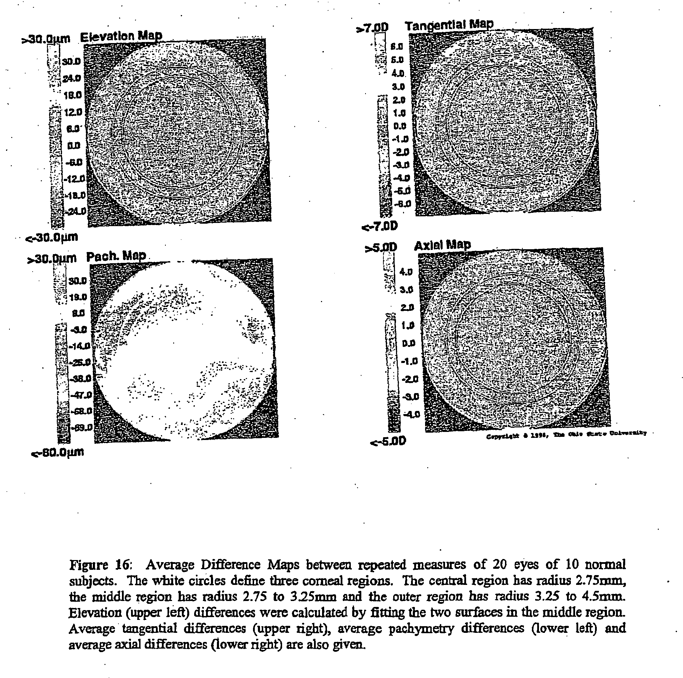

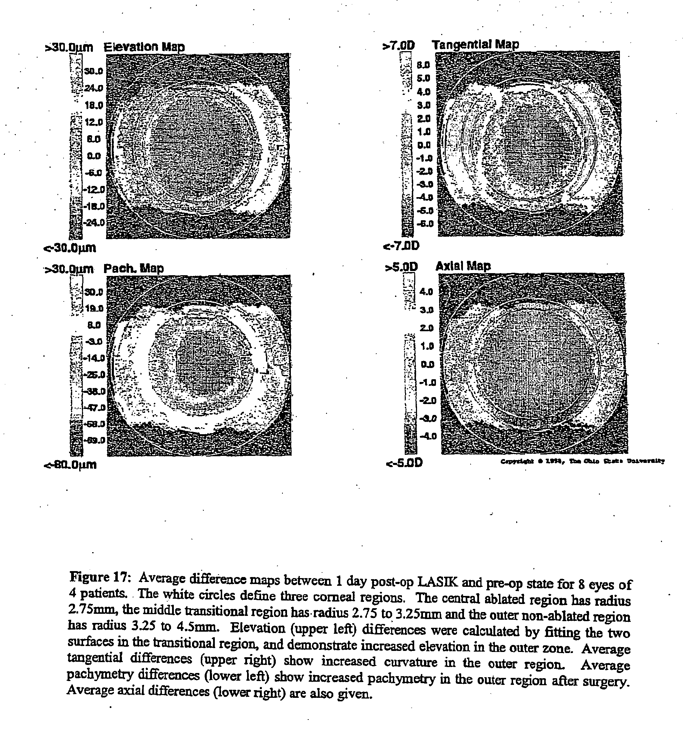

**Figures 16 and 17** show the composite difference maps of all subjects for the normal and 1 day post-operative surgical groups, respectively. Significant (p< 0.05) decreases in elevation, pachymetry, and curvature in the central zone between normals and surgical subjects were demonstrated. In addition, significant (p< 0.05) *increases* in elevation, pachymetry, and curvature in the outer zone were found, as predicted by the proposed biomechanical model. These increases persisted at 1 month post-operatively, as seen in

### Figure 18.

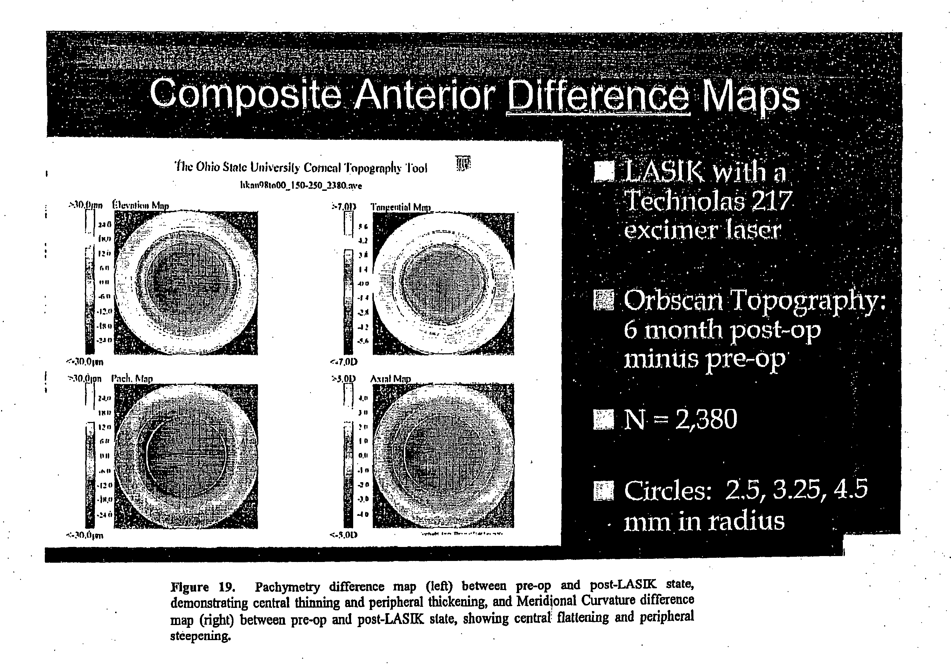

A large retrospective study was conducted of 2,380 patients who received LASIK using a Technolas 217 laser, who had Orbscan Corneal Topography pre-operatively and at 6 months post-operatively. **Figure 19** shows the composite difference maps generated from the Orbscan data, demonstrating the same pattern of peripheral increases in curvature, elevation, and thickness over a large population. In addition, central curvature difference was highly correlated with peripheral elevation difference in a regression analysis (R² = 0.76, p< 0.0001), consistent with the proposed biomechanical model of corneal response.

### In Vitro Studies

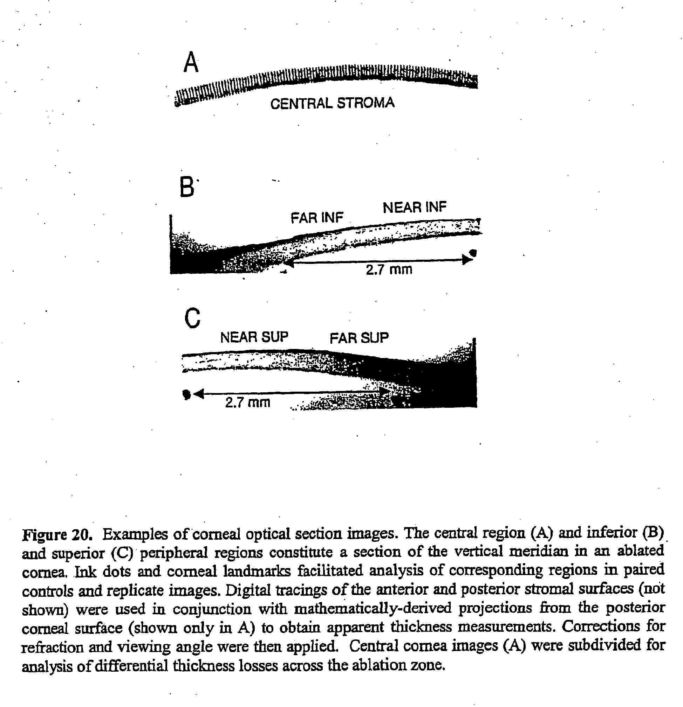

Two *in vitro* studies were conducted. In the first study, 14 deepithelialized eye bank globes from 7 donors were subjected in paired-control fashion to either broad-beam PTK (-100-um depth, no programmed dioptric change) or sham photoablation. Changes in anterior curvature were measured by autokeratometry. Changes in stromal thickness in the vertical meridian were measured using corneal optical section image analysis. This is a technique using a modified endothelial camera to obtain cross-sectional corneal images, an example of which is shown in **Figure 20**. The corneal cross-section was divided into 5 regions for analysis, far superior, near superior, central, near inferior, and far inferior. Analysis included evaluating peripheral thickness changes and geometric bias as predictors of curvature change. Geometric bias was defined as either a myopic or hyperopic bias in the pattern of ablation zone thickness loss in order to investigate current shape-subtraction theories of hyperopic shift in PTK.

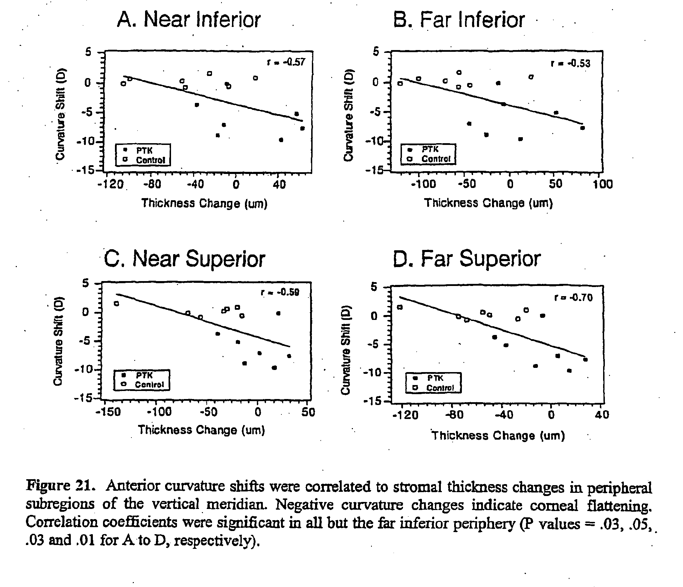

Results of this first study demonstrated that photoablation caused significant reductions in keratometric curvature (-6.28 ±3.23 D, P = .002) relative to untreated paired controls. The mean keratometric shift measured during sham PTK was not significantly different from zero (+0.31 ± 0.85 D, P = .38). In addition to dramatic flattening of the spherical component of curvature, ablated corneas demonstrated significantly higher absolute magnitudes of keratometric cylinder (2.98 ± 0.88 D) than controls (0.46 ± 1.72 D,P = .009), indicating a potential biomechanical component to induced cylinder, potentially due to nonuniform distribution of corneal crosslinking. The relative peripheral stromal thickness change, expressed as the mean pairwise difference (PTK-control), was +57.3 ±42.8 um (P = .01) or +8.5 ± 5.7 % (P = .O1), demonstrating significant thickening relative to controls and supporting the proposed new theory. Central curvature shifts were linearly dependent upon regional peripheral stromal thickness changes in ablated and control eyes (**Figure 21**). Correlations were significant in 3 of the 4 peripheral subregions and marginally significant in the far inferior subregion (P = .05). The far superior stromal subregion exhibited the strongest correlation to hyperopic shift (r = -0.70). Correlation coefficients are negative and indicate a tendency for corneas to flatten more extensively with increasingly positive peripheral thickness changes, a result that is consistent with the proposed role of peripheral thickening as a mechanical stimulus for central flattening. The correlation between keratometric shift and the mean total peripheral stromal thickness change (the mean change over all 4 subregions) was significant (r = -0.67, P = .01), and equivalent results were observed when thickness changes were expressed as percent changes.

On the other hand, calculated geometric bias favored flattening in only 50% of cases, despite the induction of hyperopic shift in all ablated corneas. In addition, mean geometric biases in ablated corneas and paired controls were not significantly different from zero (P = 0.66 and 0.52, respectively) or from each other (P=0.74). In sharp contrast to the apparent relationship between increasing peripheral stromal thickness and decreasing central curvature, geometric bias demonstrated an absence of any linear relationship with anterior corneal flattening (r = 0.06, P = .85). Multiple transformations of the data were investigated and yielded no demonstrable correlations, challenging current shape-subtraction theories of hyperopic shift in PTK. The bottom line was that geometric bias was a poor lone predictor of anterior corneal flattening. The results of this study demonstrate unequivocally that epithelial hyperplasia and stromal remodeling are not absolute requirements for the production of clinical magnitudes of anterior flattening in PTK.

A second in vitro study was performed to further examine the relationship between peripheral stromal thickening and central flattening. Specifically, a paired-control human donor eye study (n=20) was conducted to assess the efficacy of preoperative topical glutaraldehyde (GTA) treatment as a technique for inhibiting PTK-induced peripheral stromal thickening and, secondarily, for attenuating the acute corneal flattening response. Each eye was individually mounted in a custom holder, inflated to normal intraocular pressure (15 mmHg) and deepithelialized. According to a crosslinking protocol developed in preliminary experiments, one cornea of a given donor pair was immersed in a 15% dextran solution for 40 minutes then transferred to 4% GTA/dextran for an additional 20 minutes; the fellow control was exposed to 15% dextran for 60 minutes. Each eye was subsequently subjected to 1) sham PTK, a same-eye control phase incorporated to account for thinning due to intraoperative dehydration, 2) PTK (5-mm-diameter, 100um-depth) and 3) a 1-hour hypo-osmotic soak phase designed to assess the anti-swelling activity of stromal crosslinking. A scanning-slit topography system (Orbscan) was used to acquire triplicate thickness and curvature measurements before and after each experimental phase. Crosslinking significantly inhibited peripheral stromal thickening during PTK and postoperative hypo-osmotic immersion. In addition, during PTK, crosslinked corneas demonstrated 36% less hyperopic shift relative to paired controls (p = .001). The magnitude of this latter effect was linearly dependent upon the magnitude of crosslink-mediated suppression of the peripheral thickening response to PTK (r = 0.68, p =.03). The results demonstrate that acute hyperopic shifts in a donor model of PTK can be significantly reduced through preoperative application of a collagen crosslinking reagent and therefore support the conclusion that mechanical events in the corneal periphery play an important role in keratectomy - induced central curvature changes. This mechanism of curvature change is not accounted for in current surgical algorithms, and potentially plays a role in the variability in outcomes.

### Optical Coherence Tomography:

Optical coherence tomography (OCT), is an imaging technology which has only appeared in the literature since the 1990s. OCT images are analogous to ultrasound images, in that they are two dimensional cross sectional images depicting tissue reflectivity. However, in the case of OCT, the images show infrared reflectivity and demonstrate a 10 micron longitudinal resolution, as compared to the 50 micron longitudinal resolution associated with traditional ultrasound. While high frequency ultrasound does allow a 20 micron longitudinal resolution, it cannot penetrate more than 4 mm beyond the cornea. The OCT also is also limited to 3 mm penetration. However, the optically clear nature of the eye allows the retina to be imaged to a depth of 3mm. For corneal imaging, the OCT offers additional advantages in that it is non-contact, unlike high frequency ultrasound which requires a water bath and topical anesthetic.

The principles by which OCT works are those of interferometry. The OCT is essentially a Michelson interferometer with the subject's eye placed at the end of one of the light paths.

### Prediction of Biomechanical Response via Corneal Pertubation and Biomechanical Modeling

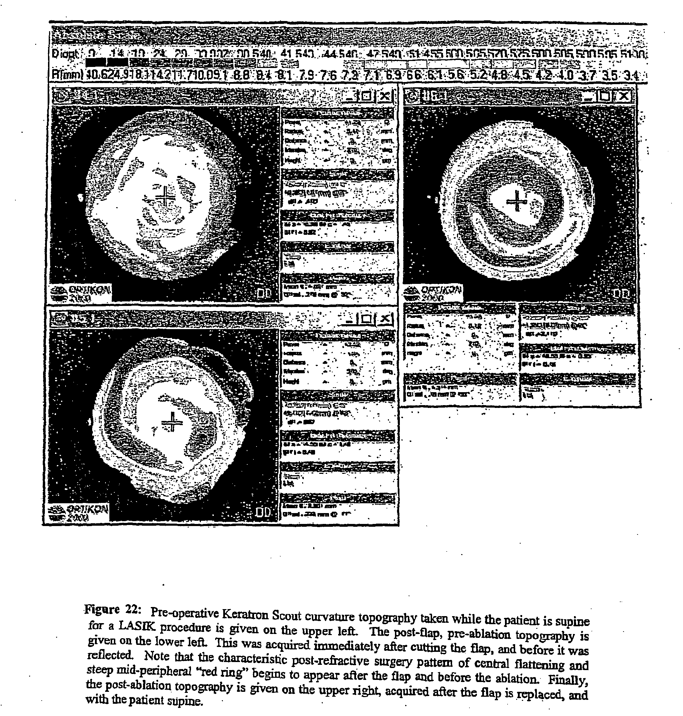

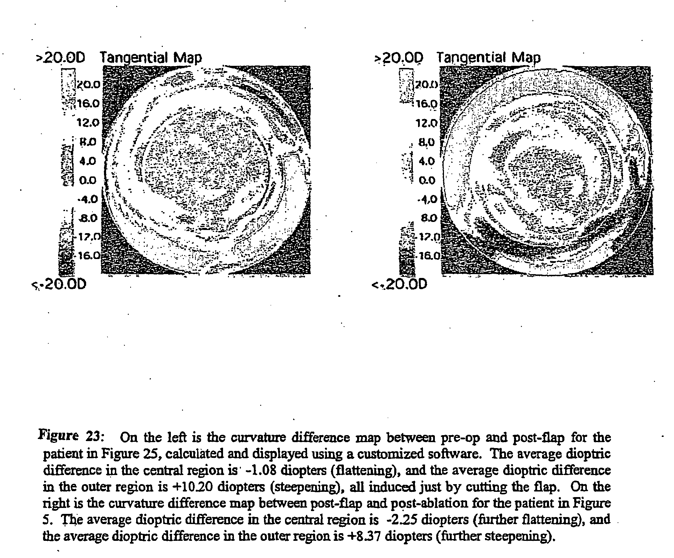

Most recently, in cooperation with two surgeons, we have measured the shape of the LASIK flap using a Keratron Scout, which is a portable, Placido-based topographer. It can be used in a vertical position intra-operatively to acquire the topography of the flap immediately after it is cut, before it is reflected, and thus prior to ablation. **Figure 22** demonstrates that the characteristic "red ring" on this patient is a biomechanical phenomenon, since it appears *BEFORE* any tissue is removed. Cutting the flap alters the corneal structure in the same way described by the model presented. The difference is that the severed lamellae are not ablated, but put back in place. Therefore, the stability of the greater than 1 diopter average central decrease in curvature shown by the difference map in **Figure 23**, which is generated simply by cutting the flap, is not known. Investigations are currently underway.

Corneal measurements following the cutting of the corneal flap (or other perturbation), but before ablation, are a key factor in embodiements of this invention. As demonstrated above, the microkeratomic incision for the flap produces definite changes in the cornea, regardless of any subsequent removal of tissue. The redistribution of strain caused by the keratomic incision causes the central cornea to flatten and the peripheral stromal matrix to thicken and become steeper. This reshaping assists with a myopic correction, i.e., a correction where increased corneal curvature is prescribed, and works against a hyperopic correction.

In one embodidment of practicing this invention, corneal topography, optical coherence tomography, ultrasound, refraction and/or wave front analysis is used both before and after the microkeratomic incision for the corneal flap (or other corneal perturbation), from a statistically sufficient number of subjects who may be surgical patients, and the differences compared to expected and achieved post-operative results (undercorrected or overcorrected). Once developed, comparison of the measurements before and after the incision will allow one to adjust the ablative procedure to account for the predicted biomechanical response, as a function of the changes measured after cutting the flap (or other corenal perturbation).. Preferably, these adjustments are made by laser manufacturers to their laser algorithms. However, since existing laser algorithms do not take the biomechanical changes into account, this invention may also be practiced by developers of corrective tables for current laser systems, or by surgeons using such tables. Ablation profile adjustments can be made in advance of the ablation in a separate procedure, or in real-time as an intra-operative adjustment, after the pertubation, but before the ablation.

As mentioned above, optimal surgical procedures also require consideration of the biomechanical results of the ablative procedure itself. This is best performed by:
(1) taking preoperative measurements;
(2) taking post-pertubation measurements after the microkeratome cut (or other corneal perturbation), but before the ablative procedure;
(3) comparing the difference between the pre-operative and post-perturbation measurements; and
(4) making appropriate adjustments in the ablative algorithm based on the magnitude and pattern of the post-pertubation response; and
(5) taking additional measurements after the ablative procedure to document the results. In a second embodiment of practicing this invention, corneal topography, optical coherence tomography, ultrasound, refraction, and/or wave front analysis are acquired both pre-operatively and post-operatively, after the laser refractive procedure, from a statistically sufficient number of subjects who are surgical patients. The pre-operative data are used to partially define the specifications of individual biomechanical mathematical models of individual corneas (thickness profile, curvature profile, corneal size). The ablation profile with which an individual was treated, is then used to mathematically "remove" layers from the model, and the post-operative data are used to define the final condition of the model. PRK, LASIK, and LASEK would each require distinct mathematical models, since the tissue removed is distinctly located within the corneal stroma. (e.g. PRK and LASEK are surface ablations and LASIK is a deeper ablation.) Knowledge of the pre-operative and post-operative state, measured from the subject, allows the model properties (e.g. Young's modulus) to be adjusted in_order to match the final predicted post-operative state achieved by the model with the actual measured post-operative state, in an iterative process. The material properties thus determined for all corneas and all models within the subject population will be correlated with the pre-operative population data (e.g. age, sex, race, years of contact lens wear) and measured pre-operative data (e.g. thickness, curvature, wavefront, corneal size), to determine which pre-operative parameters (both characteristic and measured) best predict the material properties and thus response. These correlations will be used to compile a program that will produce material properties as an output, with pre-operative data as input Once developed, the material properties program, along with individual pre-operative data, will be used to fully define a predictive biomechanical model of individual response to corneal ablation. Model predictions will allow one to adjust the ablative procedure to account for the predicted biomechanical response. Preferably, these adjustments are made by laser manufacturers to their laser algorithms. However, since existing laser algorithms do not take the biomechanical changes into account, this invention may also be practiced by developers of corrective tables for current laser systems, or by surgeons using such tables.

As mentioned above, optimal surgical procedures also require consideration of the biomechanical results of the ablative procedure itself. This is best performed by:
(1) taking preoperative measurements;
(2) predicting the biomechanical properties based on pre-operative data;
(3) fully defining a predictive biomechanical model based on pre-operative data and predicted material properties; and
(4) making appropriate adjustments in the ablative algorithm based on model predictions; and
(5) taking additional measurements after the ablative procedure to document the results.

In a third embodiment of practicing this invention, corneal topography, optical coherence tomography, ultrasound, refraction, and/or wave front analysis are acquired pre-operatively, both before and after the microkeratomic incision for the corneal flap (or other corneal perturbation), and post-operatively, after the laser refractive procedure, from a statistically sufficient number of subjects who are surgical patients. The pre-operative data and are used to partially define the specifications of individual biomechanical mathematical models of individual corneas (thickness profile, curvature profile, corneal size). The ablation profile with which an individual was treated, is then used to mathematically "remove" layers from the model, and the post-operative data are used to define the final condition of the model. PRK, LASIK, and LASEK would each require distinct mathematical models, since the tissue removed is distinctly located within the corneal stroma. (e.g. PRK and LASEK are surface ablations and LASIK is a deeper ablation.) Knowledge of the pre-operative, and post-operative state, measured from the subject, allows the model properties (e.g. Young's modulus) to be adjusted in order to match the final predicted post-operative state achieved by the model with the actual measured post-operative state, in an iterative process. The material properties thus determined for all corneas and all models within the subject population will be correlated with the pre-operative population data (e.g. age, sex, race, years of contact lens wear), measured pre-operative data (e.g. thickness, curvature, wavefront, corneal size), as well as the post-pertubation data, to determine which pre-operative parameters (both characteristic and measured) and/or post-pertubation parameters best predict the material properties and thus response. These correlations will be used to compile a program that will produce material properties as an output, with pre-operative and post-pertubation data as input. Once developed, the material properties program, along with individual pre-operative data and post-pertubation data, will be used to fully define a predictive biomechanical model of individual response to corneal ablation. Model predictions will allow one to adjust the ablative procedure to account for the predicted biomechanical response, based on pre-operative and post-pertubation data. Preferably, these adjustments are made by laser manufacturers to their laser algorithms. However, since existing laser algorithms do not take the biomechanical changes into account, this invention may also be practiced by developers of corrective tables for current laser systems, or by surgeons using such tables. Ablation profile adjustments can be made in advance of the ablation in a separate procedure, or in real-time as an intra-operative adjustment, after the pertubation, but before the ablation.

As mentioned above, optimal surgical procedures also require consideration of the biomechanical results of the ablative procedure itself. This is best performed by:
(1) taking preoperative measurements;
(2) taking post-pertubation measurements after the microkeratome cut (or other corneal perturbation), but before the ablative procedure;
(3) predicting the biomechanical properties based on pre-operative data and post-pertubation data;
(3) fully defining a predictive biomechanical model based on predicted material properties, pre-operative data; and post-pertubation data
(4) making appropriate adjustments in the ablative algorithm based on model predictions; and
(5) taking additional measurements after the ablative procedure to document the results.

These processes may be performed most effectively with laser manufacturers. They have already made empirical adjustments to the algorithms originally proposed by Munnerlyn et al, and should be eager to make further adjustments that would produce superior results. Similarly, the results of the corneal healing process should be considered. This is another iterative process taking measurements immediately following an operation and at specified intervals thereafter to determine biomechanical modifications in the cornea following surgery. This is also applicable to customized ablation, based on topography-guided or wavefront-guided procedures that do not rely on the Munnerlyn formulas.

The corneal healing response is characterized using OCT imaging to measure epithelial, stromal, and flap thickness. For each examination, five images are acquired in the vertical meridian and five in the horizontal meridian, until the remote site completes the software to scan the cornea in a radial spoke pattern covering the central cornea within an 8mm diameter. The OCT examinations is performed at the same time points as the topographic measurements for comparison purposes: pre-operatively and post-operatively at one day, one week, one month, three months, and six months, with the addition of one time point immediately after surgery in order to visualize the flap interface.

The biomechanical response is believed to be immediate, with modification through the healing phase. Therefore, the behavior of the model is defined based on one day, one week, one month, 3 months, and 6 months post-operative measurements, with the exception of the OCT measurements that will be taken immediately after the surgery in order to visualize the flap. Once the model is fully characterized, and performance is satisfactory, an optimization approach is used to design new ablation algorithms. Rather than providing an ablation profile to the model and predicting the corneal response, optimization criteria is used as input to produce the necessary ablation profile to meet the criteria. Examples may be to maximize ablation zone diameter, while minimizing ablation depth to reach a target central curvature with minimal aberrations.

Post-operative corneal shape, and thus visual performance, is the function of at least three factors: the ablation profile, the healing process, and the biomechanical response of the cornea to a change in structure. Only by increasing our knowledge of the interaction of these factors can predictability in PRK and LASIK be improved. This has important implications in the development of new ablation algorithms and guided procedures. It points to an optimization approach, rather than a priori defining an "ideal" corneal shape that is ultimately not achievable. There are only certain shapes a cornea will biomechanically assume. For example, the deeper the peripheral cut in a myopic procedure to generate a potentially desirable post-operative "prolate" shape, the greater the number of severed lamellae and the greater the biomechanical central flattening response to counter the effect. *Both* the ablation profile *and* the biomechanical response need to be taken into account, as well as the healing response. Therefore, step one is to gain a better understanding of corneal response to standard ablation profiles in well-controlled studies, *before* moving into the realm of customized procedures. The outcome measures in these controlled studies should be more comprehensive than in the past to allow us to thoroughly interrogate the corneal response. This means we need to measure and report the outcome error in terms of the predicted topography and/or the predicted wavefront, not just visual acuity, spheres and cylinders.

### Sample Finite Element models demonstrating the unique attributes of the invention

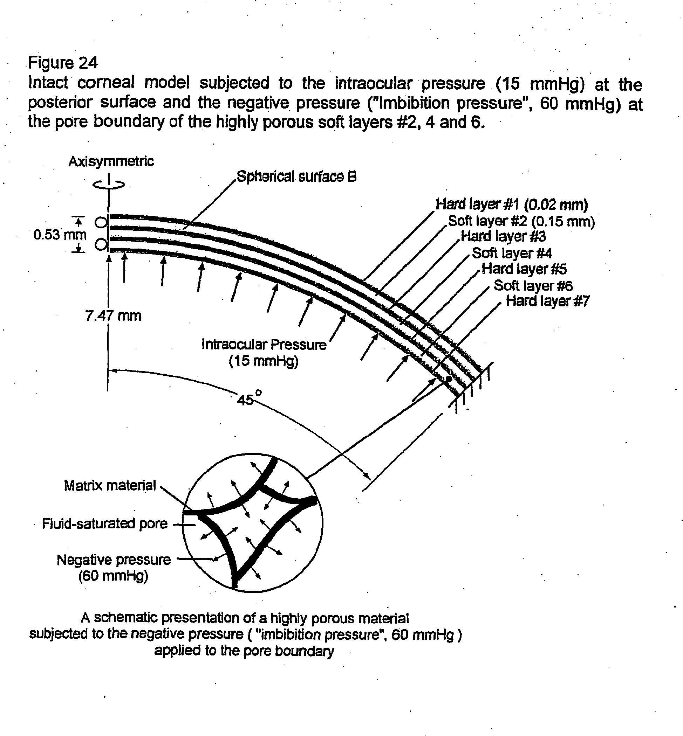

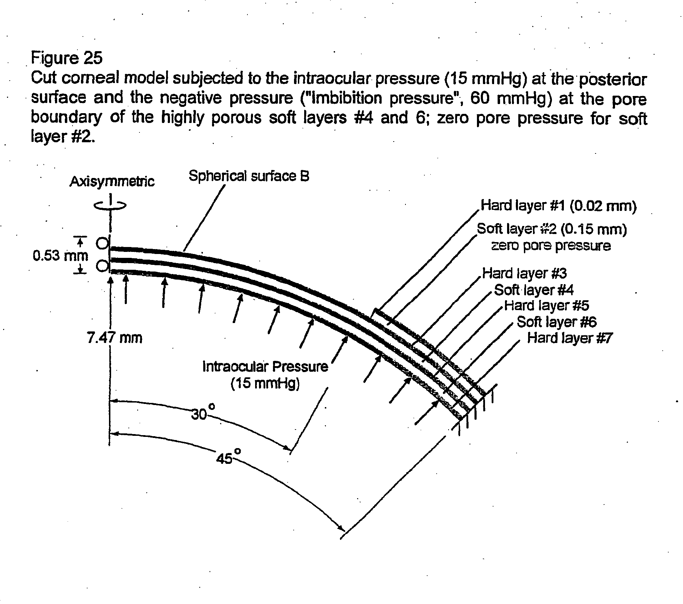

Major advantages of this invention are obtained by directly introducing the negative ("imbibition") pressure into the simulations with simple models as shown in **Figures 24 and 25**. In **Figure 24**, a model for intact cornea is presented as a portion of an axisymmetric layered spherical shell fixed at the ends. This model consists of alternately, placed four thin hard layers (layer #1,3,5,7) and three thick soft layers (layer #2,4,6). In this simplified model, the collagen fibers are schematically modeled as impermeable hard layers. Soft layers, which are assumed to be highly porous and fully saturated with water, representing the matrix or ground substance containing proteoglyeans.

As discussed before, the major portion of the actual cornea (stroma) consists of over 300 ~ 500 layers throughout the thickness and each layer is reinforced by a number of long collagen fibers with the same direction. The direction of the collagen fibers in each layer is different so that the in-plane reinforcement is more or less uniform. The model is simplified not only because the number of layers is reduced from 300~500 to 7 but also because the collagen fibers are collectively assumed to be thin hard shell layers instead of maintaining individual long cylindrical shape. This simplified model, however, is significantly different from any existing corneal finite element model in the sense that the basic microstructure of the cornea as a layered or lamellar structure, along with being highly porous, is directly incorporated into the finite element model. Iii addition, basic substances which make up the cornea, such as collagen fibers and ground substance (proteoglycans) and water are modeled separately. In **Figure 25**, a simple model for an ablated cornea after surgery relevant to PRK is presented. A part of the two outer layers (layer #1 and #2) is removed from the intact corneal model.

**TABLE 3: Layer Geometry and Material Properties**

| Layer number | #1,3,5,7 | #2,4,6 |
|---|---|---|
| | (Hard) | (Soft) |
| Thickness [mm] | 0.02 each | 0.15 each |
| | (4 layers) | (3 layers) |
| Young's modulus * | 5x10⁸ | 4x10⁴ |
| E [pa] | | |
| Poisson's ratio** | 0.3 | 0.3 |
| ν | | |

| | | |
|---|---|---|
| * J.O. Hjortdal, Regional Elastic Performance of the Human Cornea, Journal of Biomechanics (1996) 29, 931-942. | | |
| **T.J. Shin, R.P. Vito, L.W. Johnson and B.E. McCarey, The Distribution of Strain in the Human Cornea (1997) 30, 497-503. | | |

In the above simple models, the overall material properties for both hard and soft layers are assumed to be linearly elastic and isotropic. The elastic moduli are taken from the' literature. As discussed before, the soft layer is assumed to be a highly porous material. Based on the existing theories for fluid-filled porous materials (Katsube, 1985; Katsube and Carroll, 1987a,b), this porous material assumption requires additional elastic material parameter such as the bulk modulus of the solid matrix material of the soft layer without pores. The physical measurement of the bulk modulus of the matrix itself (without pores) will be extremely difficult and has not been reported in literature. In the simple models, this bulk modulus is assumed to be infinite, and the matrix itself (without pores) is assumed to be incompressible. This assumption is reasonable because the volume fraction of the matrix is extremely low in the cornea, and much of the volume compaction or expansion of the soft layers can be attributed to the pore space compaction or expansion through the deformation of the matrix.

The volumetric expansion of a porous solid material due to the internal pore pressure is similar to volumetric thermal expansion due to temperature increase although the physical mechanisms are completely different. Therefore, this volume change due to the internal pore pressure can be replaced by the thermal expansion term in any of the commercially available finite element codes, provided the material properties of the (dry) porous material (without fluid) and those of the solid matrix (without pores) are known.

As shown in **Figure 24**, the negative ("imbibition") pressure of magnitude 60 mm Hg is applied uniformly throughout the pore boundary of highly porous layers #2, #4, and #6, in addition to the intraocular pressure of magnitude 15 mmHg applied on the posterior surface of the cornea. In carrying out the simulations, the commercially available finite element code ABAQUS with four-node axisymmetric elements is employed. In Figure NK2, the negative ("imbibition") pressure is applied only to porous layer #4 and #6, and zero pore pressure is assumed in the centrally ablated or cut porous layer #2. This simplified assumption is based on the fact that the fluid movement is most likely to occur in the in-plane direction rather than out-of-plane direction due to the layered nature of actual cornea. Since the internal fluid is suddenly exposed to the atmospheric pressure at the place where the layer is severed, the internal pressure is set equal to zero in layer #2.

### Results based on the sample finite element models

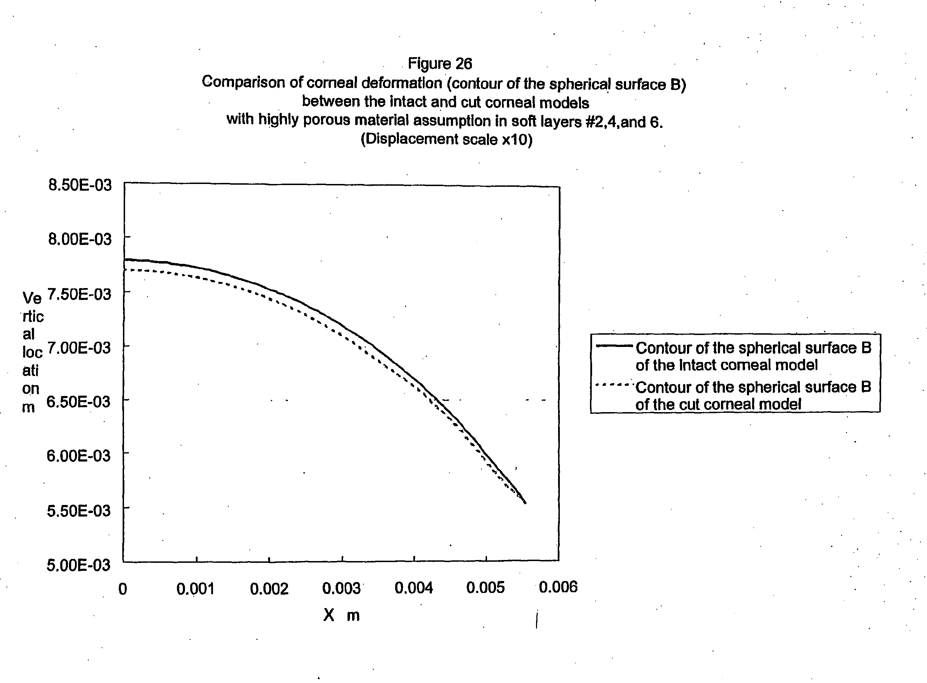

The contours of the spherical surface B (the interface between the soft layer #2 and the hard layer #3) for the intact cornea model (**Figure 24**) and ablated or cut cornea model (**Figure 25**) are plotted in **Figure 26**. The displacements are magnified by a factor of 10 so that the comparison before and after the surgery can be visually observed. The spherical surface B flattens after the surgery. This flattening due to the surgery demonstrates the same trend as the conceptual model, supported by clinically observed results described earlier in this application.

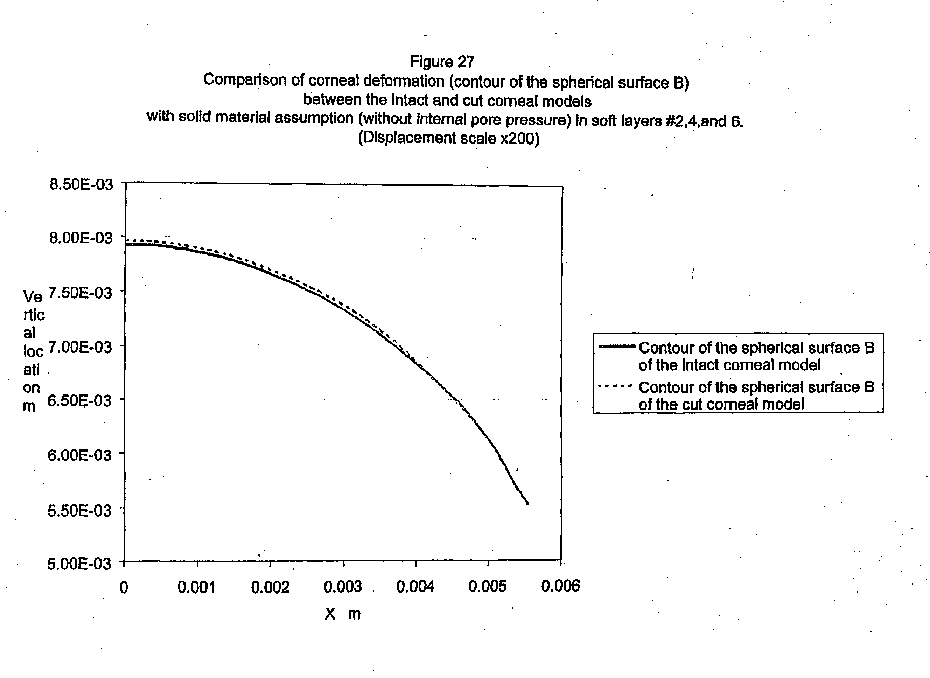

As discussed previously, in all the existing methodologies of refractive surgeries, the fact that the cornea is a porous material is ignored. Instead, the cornea is treated as a solid material. Therefore, the negative ("imbibition") pressure applied through the internal pore boundary inside the cornea is not taken into account. In order to elucidate these differences, we plot the deformation of the spherical surface B before and after the surgery by setting negative "imbibition" pressure in soft layers #2, #4, and #6 equal to zero. As shown clearly in **Figure 27**, the spherical surface B bulges after the surgery. This is not consistent with clinical results of surgery. The bulging occurs because the central part of the cornea has less rigidity due to the removal of tissue and therefore deforms more after the surgery.

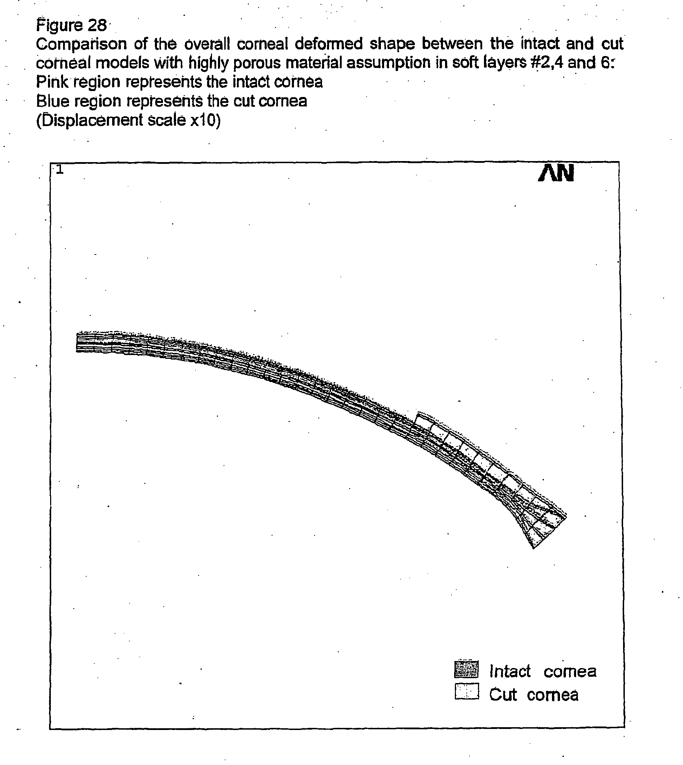

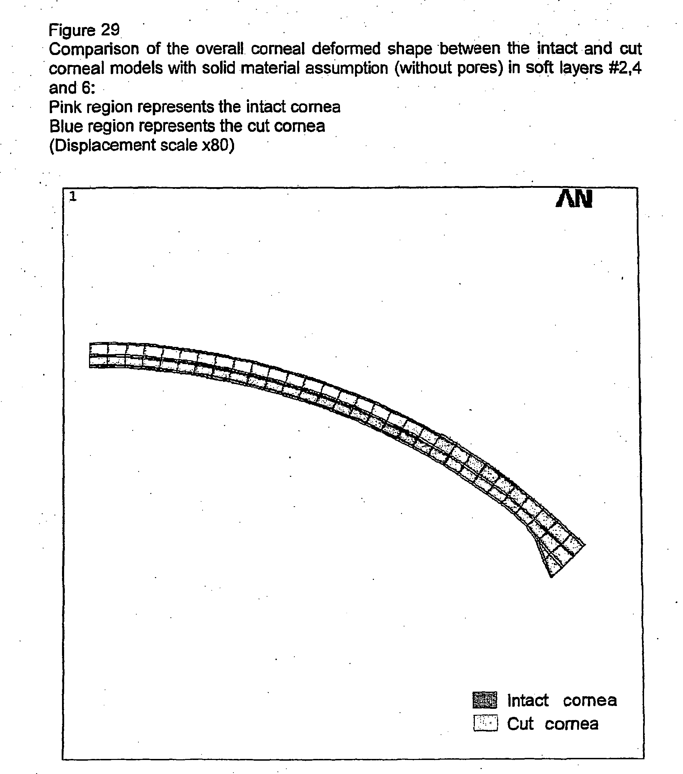

In order to demonstrate further the differences between existing simulations and simulations based on the invention, the overall corneal deformed shapes before and after the surgery are compared based on the two different approaches. In **Figure 28**, the overall corneal deformed shapes for the intact and ablated or cut corneal models are compared based on the fluid-filled porous material assumptions for soft layers. The peripheral portion of the cornea after ablation is thicker than that before ablation. This peripheral thickening matches the conceptual model supported by clinical results, described earlier in this application. In **Figure 29**, the overall corneal deformed shapes between the intact and ablated or cut corneal models are compared based on the solid material assumptions for soft layers. As opposed to the results based on the fluid-filled porous material assumption shown in Figure 28, the peripheral thickening after ablation is not observed. Therefore, the existing simulations based on the solid material assumption are not consistent with clinical results after laser refractive surgery, described in this application.

An ablation depth dependent biomechanical response was described, such that more ablation implies more flattening. Preliminary calculations are performed for a two-set layer ablation where a part of the layer #1,2;3,4 (instead of #1 and #2 as in Figure NK2) is ablated or cut. Based on the fluid-filled porous material assumption for soft layers, more flattening is observed. However, based on the solid material assumption for soft layers, more bulging is observed. This is because the structure rigidity of the central portion of cornea is further eroded due to further thinning. Therefore, the clinical trend supported in this application is reproduced based on the simulations based on the invention, while the wrong trend is reproduced by simulations based on the existing methods.

In addition, current age nomograms for myopic procedures generally tend to require less ablation with increasing age, to achieve the same level of correction. It is also known that the corneas of older individuals tend to be stiffer than those of younger individuals. Preliminary parametric studies based on the simple finite element models presented here demonstrate that when Young's modulus (layer #1,3,5,7) of the fiber is reduced by a factor of 10, less flattening in the central portion of the corneal surface is observed. Therefore, the trend of basic age nomograms for myopic procedures are reproduced with this innovative model.

The importance of this invention is demonstrated through these simple sample models. Without the direct incorporation of the negative ("imbibition") pressure inside the cornea into the simulation, the very basic clinically known results relevant to LASIK or PRK cannot be properly modeled.

### Extension of the sample finite element models

In some of the existing simulations of refractive surgeries, the swelling due to the negative "imbibition" pressure in the cornea is indirectly discussed in terms of "hydration" (Roy et al. 1996). However, the clear notion that the cornea is highly porous is not directly modeled into the formulation. Therefore, there is no separation between the fluid and the solid constituents inside the cornea, and the cornea is treated as a "hydrated" solid material. Direct incorporation of negative ("imbibition") pressure and the position (and time)-dependent change of this pressure due to refractive surgeries into the formulation have never been reported in literature.

This leads to the fact that the key part of this invented modeling can be easily extended to all the other existing simulations of refractive surgeries (R. P. Vito, T. J. Shin, and B. E.. McCarey, "A Mechanical Model of the Cornea: The Effects of Physiological and Surgical Factors on Radial Keratotomy Surgery," Refractive & Corneal Surgery, 1989, p. 82-88. P. Pinsky and D. V. Datye, "A Microstructrurally-Based Finite Element Model of the Incised Human Cornea," Journal of Biomechanics, vol. 24, 1991, p. 907-922. P. Pinsky and D V. Datye, "Numerical Modeling of Radial Astigmatic, and Hexagonal Keratotomy, Refractive & Corneal Surgery, vol. 8, 1992, p. 164-172. K. D. Hanna, F. E. Jouve, G. O. Waring, and P. H. Ciariet, "Computer Simulation of Arcuate Keratotomy for Astigmatism," Refractive & Corneal Surgery, vol. 8, 1992, p. 152-163. P. Roy, W. M. Petroll, A.E. McKinney an C.J. Chuong, "Computational Models of the Effects of Hydration on Corneal Biomechanics and the Results of Radial Keratotomy, Journal of Biomechanical Engineering, Transactions of the ASME, vol. 118, 1996, p. 255-258. M. R. Bryant, V. Marchim T. Juhasz, ''Mathematical Models of Picosecond Laser Keratomileusis for High Myopia," Journal of Refractive Surgery, vol, 16, 2000, p, 155-162). As discussed before, in existing simulations, the cornea is treated as a homogeneous "hydrated" solid material. All we need to do is to make clear throughout the formulation that this homogeneous "hydrated" solid material is in fact an "equivalent" homogeneous solid material, and this "equivalent" homogeneous solid material is actually highly heterogeneous in the sense that it contains pores and pores are filled with water.

Conceptually speaking, porosity (pore volume per unit volume of a porous solid material) can be introduced at each point of this equivalent homogeneous material, and the deformation due to the internal pore pressure, which is missing in the existing methods, can be considered. Theoretically speaking, the volume expansion due to internal pore pressure can be simulated by mimicking thermal expansion due to temperature change. Given the specific value of pore pressure inside the cornea, this replacement can be done by knowing the overall material properties of the porous solid material (solid matrix and pore) and those of the solid matrix (without pores). As is explained in the example models, since the cornea is highly porous, much of the overall volume change can be attributed to the change in pore space rather than the expansion or compaction of the solid matrix (without pores). Therefore, by employing the simplified assumption of incompressibility of the solid matrix (without pores), the expansion due to the given pore pressure can be simulated through thermal expansion terms. This leads to the direct incorporation of the negative ("imbibition") pressure applied to the pore boundary of the highly porous cornea. In this way, the existing simulations can be modified to include the effect of the position-dependent negative ("imbibition") pressure inside the cornea on the corneal biomehanical response to the surgery.

In the simple example models, linearly elastic and isotropic material assumption is employed. However, the material response assumption can be extended to anisotropic and/or nonlinear models. Therefore, by incorporating the basic idea of the existing theories for fluid-filled porous materials into the existing simulations, we can significantly improve the existing simulations for all other refractive surgeries.

In the simplified example models, the hard layer representing the collagen fibers and the soft layer representing the matrix or ground substance and water are modeled separately. However, this layered structure can be modeled as an "equivalent" homogeneous anisotropic material on the basis of the composite material models for layered structure by Katsube and Wu ("A Constitutive Theory for Porous Composite Materials," International Journal of Solids and Structures, Vol. 35, pp. 4587-4596, 1998). By introducing the volume fraction of each layer (thickness ratio) and the porosity at each point of an "equivalent" homogeneous material, deformation of the fiber and the ground substance and water inside the cornea can be separated within the framework of a single, continuum model. This homogenization technique can again be used as a tool to introduce more detailed micro-structure to the existing simulations. If the detailed information regarding the microstructure is introduced in the modeling; more detailed information can be obtained from simulations. In addition to the above analytical methods, these processes of introducing more microstructure into modeling can also be achieved by simply carrying out several levels of macro-micro numerical simulations. For example, in the smallest scale level, local positiorial arrangement (orientation) of individual collagen fibers may be modeled. In addition, the reorientation and stretch of collagen fibers as well as undulation structure of individual collagen fibers can also be separately modeled. These small-scale level models can be incorporated into the intermediate-scale level of layered structural model. The obtained intermediate-scale level model can further be incorporated into the large-scale boundary value problems of corneal deformation.

The example models are used simply to demonstrate the key ideas of this invention. The same idea can be extended to include various other factors such as time dependent movement of fluid and electrochemical considerations. Initial boundary value problems of fluid flow can be added to the model simulations so that the time-dependent nature of corneal deformation can be modeled. In addition to the above purely mechanical consideration of cornea, electrochemical balance as well as ion transport can be added to the model simulation. The ion and fluid transport phenomena are coupled and both influence the deformation of cornea. In both cases, the direct incorporation of negative ("imbibition") term becomes very important in solving initial boundary value problems since the atmospheric pressure that appears at the ablated or cut surface can be specified as boundary conditions.

The simple finite element models based on this invention can predict the correct trend in corneal response to laser refractive surgery, as opposed to existing models in the literature. With minor adjustments in geometry and microstructure, the simple models used to describe this invention are immediately useful in predicting the general trend in outcomes of refractive procedures.

In a LASIK procedure, a flap is first created by a microkeratome and then corneal ablation is performed. After ablation, the hinged flap will be replaced in its original position. Upon further refinement of the simulations based on this invention, material properties relevant to the model can be back calculated from the biomechanical response of a cornea due to the flap creation (but before ablation). The mechanical response due to ablation can then be simulated and the post-operative condition can be predicted based on the model simulations. This information can assist the physicians to make decisions about the operation before actual ablation. Therefore, this invention can be very useful in increasing the success rate of refractive surgery.

### Integrated System for Refractive Surgery

The modeling, optimization and algorithm correction of the present invention may be achieved by written calculation or by implementation via a computer system. Those skilled in the art of mathematical computer modeling, computer programming, and database manipulation and administration will readily appreciate that the methods and systems described herein may be software based and may be performed by a computer system.

For example, a computer comprising a central processing unit, a storage medium (such as a magnetic hard drive), a display device (such as a monitor) and an input device (such as a keyboard and/or a mouse) may be used to perform the methods of the present invention. Pre-operative and post-pertubation measurements are inputted to the computer system by keystroke or by other similar methods, including direct data transfer of measurements inputted into another computer or similar machine. Existing algorithms for ablative procedures may be inputted in digital form by any appropriate method. Additional empirical, deterministic or other data may be inputted to the computer by any appropriate method.

Data inputted into the computer is stored by any appropriate method, including read- only memory (RAM) or other storage devices, such as a hard disk drive.

Analysis and comparison of inputted and/or stored data items is achieved by implementing appropriate software algorithms on the computer which compare the relative values of stored data. Adjustment of existing algorithms for ablative procedures is achieved by retrieving a ablative procedure from data storage and modifying it. The modified algorithm is restored or implemented during surgery.

Data derived or created by the present invention is stored in a computer by any appropriate method, including storage on an appropriate computer readable medium. An example of an appropriate computer readable medium is RAM or a magnetic hard drive. Depending upon the nature of the data, the data may be stored in a table or other appropriate data structure on such a computer readable medium. Those skilled in the art of databases will readily appreciate that multiple types of data structures are appropriate under the present invention.

Modeling is achieved by implementing appropriate software algorithms on the computer which use stored data in conjunction with mathematical modeling algorithms. Those skilled in the art of computer modeling will readily appreciate that many appropriate software modeling applications are available and appropriate under the present invention.

A computer system implementing a method of the present invention may be used during cornea surgery. Data may be inputted to such a computer system during the surgical process, and the computer system outputs responsive data during the surgery. For example, a surgeon or other personnel at surgery inputs pre-operative measurements into the computer. The computer, implementing the present invention, determines specifications for ablation based upon such measurements. After cutting an incision or other pertubation, post-pertubation measurements are acquired by diagnostic devices (corneal topography, wavefront analyzer, optical coherence tomography, ultrasound) which are integrated with the laser to allow all measurements to share a common reference axis. These data are inputted into the computer via the integrated system. The computer determines adjusted specifications for ablation, and outputs such adjustments to the surgeon. The surgeon implements the ablation with the adjusted specifications.

A computer system implementing a method of the present invention may also be used prior to cornea surgery to create a biomechanical model based upon inputted data. A computer system implementing a method of the present invention may also be used subsequently to cornea surgery to collect data from the surgery to be used in biomechanical modeling.

### What is the Impact of Corneal Biomechanics on Customized Ablative Procedures?

The studies described above clearly show that corneal biomechanics, including the reshaping of the cornea that results from a microkeratome incision, the reshaping of both the central area of the cornea and the surrounding peripheral area that results from ablative surgery, and the reformation produced by post-operative healing must be accounted for to establish truly customized ablative procedures for individual patients. Current ablative algorithms do not adequately reflect these biomechanical factors. Thus, surgical results to date have frequently fallen short of achievable optimum goals. Fortunately, however, manufacturers of diagnostic and surgical equipment, such as corneal topographers, optical coherence tomographers, wave front analyzers and lasers, and surgeons, can account for these factors using the procedures and instruments described herein. 20/40 visual acuity with plus or minus one diopter of residual refractive air should no longer be acceptable results for LASIK regular or PRK regular surgery. 20/20 or even 20/10 visual acuity, with minimal aberration, should be achievable with most patients.

What is the bottom line for the future of customized laser refractive surgery? Can we better predict post-operative shape and visual performance? Can we reach toward 20/10 visual acuity with minimized aberrations? These are absolutely achievable goals if we unravel the controllable and/or predictable sources that contribute to the final corneal shape and visual outcome. Which instruments will be important to "guide" the laser ablation? It is highly likely that both wavefront analysis and corneal topography will be necessary to program the laser profile in order to consistently achieve an optimized result. Wavefront analysis will provide the means necessary to measure and minimize aberrations. Topography will help us measure and predict the biomechanical corneal response in ways that have not yet been elucidated. The future of customized laser refractive surgery is certainly bright. Those skilled in the art of corneal surgery, or the development of diagnostic or surgical equipment for these procedures will readily appreciate that the methods and instruments described herein can produce dramatic improvements in corneal surgery. Of course, they will also realize that many modifications may be made in these procedures and instruments, which are merely illustrative. These illustrations are not intended to limit the scope of this invention, which is defined by the following claims.

## Claims

1. An ophthalmic laser system for performing customized refractive ablation procedures, the system having means for supporting a patient in a therapeutic position, thereby establishing a reference axis via fixation point with respect to a cornea of the patient, a laser adapted to be positioned with respect to the reference axis for ablating the cornea according to an ablation specification, and at least one measuring instrument adapted to be positioned with respect to the reference axis for taking data of the cornea, said system is **characterized by**:
a data receiver in communication with the at least one measuring instrument and adapted to receive a first corneal data associated with the cornea before a corneal perturbation and for receiving a second corneal data associated with the cornea after the corneal perturbation;
a modeler in communication with the data receiver, and adapted to provide a predictive corneal biomechanical response model to the corneal perturbation based on at least the first corneal data; and
an ablation specification processor in communication with the modeler and adapted to compute the ablation specification based at least in part on the predictive corneal biomechanical response model.

2. The system of claim 1, further comprises a database containing biomechanical data from a statistically significant number of corneas before and after receiving the corneal perturbation, wherein said modeler is adapted to access said database to correlate at least the first corneal data to the biomechanical data.

3. The system of claim 1, where the ablation specification processor is further adapted to update the ablation specification based at least in part on the first corneal data, the predictive conical biomechanical response model, and the second corneal data.

4. The system of claim 1, where the first corneal data is topographic data.

5. The system of claim 1, where the first corneal data is pachymetric data.

6. The system of claim 1, where the first corneal data is elevation data.

7. The system of claim 1, where the first corneal data is total corneal thickness data:

8. The system of claim 1, where the first corneal data is corneal curvature data.

9. The system of claim 1, where the first corneal data is wave front data.

10. The system of claim 1, where the first corneal data is intraocular pressure data.

11. The system of claim 1, where the first corneal data is prior surgical response data.

12. The system of claim 1, where the first corneal data is ultrasonic data.

13. The system of claim 1, where the second corneal data is topographic data.

14. The system of claim 1, where the second corneal data is pachymetric data.

15. The system of claim 1, where the second corneal data is elevation data.

16. The system of claim 1, where the second corneal data is total conical thickness data.

17. The system of claim 1, where the second corneal data is corneal curvature data.

18. The system of claim 1, where the second corneal data is wave front data.

19. The system of claim 1, where the second corneal data is intraocular pressure data.

20. The system of claim 1, where the second corneal data is peripheral stoma thickness data.

21. The system of claim 1, where the second corneal data is ultrasonic data.

22. The system of claim 1, where the ablation specification is an ablation pattem.

23. The system of claim 1, where the ablation specification is an ablation location.

24. The system of claim 1, where the ablation specification is an ablation size.

25. The system of claim 1, where the ablation specification is an ablation depth.

26. The system of claim 1, where the ablation specification is a number of ablations.

27. The system of claim 1, where the at least one measuring instrument comprises a peripheral stromal thickening analyzer, a corneal elevation analyzer, a position dependent negative pressure analyzer, a conical lamellae severing analyzer, an acute depth response analyzer, a wave front analyzer, an ultrasonic device, a corneal topographer, an optical coherence tomographer, an autorefractor, and combinations thereof.

28. The system of claim 1, where the modeler is adapted to model the cornea as a hydrated porous structure comprising a series of hard layers separated by at least one soft porous layer, with collagen fibers modeled as one or more impermeable hard layers.

29. The system of claim 28 wherein the collagen fibers are collectively assumed to be thin hard shell layers.

30. The system of claim 1, where the modeler is adapted to model the cornea as a layered or lamellar, highly porous structure, comprising a plurality of said thin hard shell layers separated by at least one soft porous layer.

31. The system of claim 30 wherein said porous structure comprises a matrix of incompressible substance with pores inside said matrix, at least some of said pores being at least partially filled with fluid.

32. The system of claim 1, where the modeler is adapted to model the cornea as a hydrated structure of a porous material, the structure comprising a plurality of hard layers separated by one or more soft porous layers, where collagen fibers are modeled as impermeable hard layers and where the hydrated porous material is a matrix of an incompressible substance with pores inside the matrix, and where one or more pores are at least partially filled with a fluid.

33. The system of claim 1, where the modeler is adapted to model the cornea represented by the corneal data as a portion of a hydrated porous shell comprising fixed edges, a substantially uniform intraocular pressure of between about 10 mmHg to 15 mmHg against an inner surface of the shell, and a subatmospheric pressure within the shell.

34. The system of claim 1 wherein the perturbation is adapted to ultrasonically deform the cornea.

35. The system of claim 1 wherein the perturbation is adapted to create a flap on an anterior surface of a cornea.

36. The system of claim 1 wherein the perturbation is adapted to ablate a portion of a cornea.

37. The system of claim 1 wherein the perturbation is adapted to peel an epithelial layer from the cornea.

38. The system of claim 1 wherein the perturbation is adapted to mechanically deform the cornea.

39. The system of claim 1 where the perturbation is adapted to create a corneal incision.

40. The system of claim 2 where the biomechanical data comprises vision acuity data, topographic data, pachymetric data, elevation data, total corneal thickness data, corneal curvature data, wave front data, ultrasonic data, intraocular pressure data, peripheral stroma thickness data, halo effect data, patient satisfaction data, prior surgical response data, post-operative healing data, and combinations thereof.

41. The system of claim 35 wherein the second corneal data comprises a thickness measurement of the flap.

42. The system of claim 1 wherein the second corneal data comprises post-operative diagnosis data at selected post-operative increments to assess changes in the cornea in the healing process.

43. The system of claim 42 wherein said post operative increments comprise at least one of about one day, about one week, about one month, about six months, and about nine months following refractive cornea surgery.

44. The system of claim 1 where the modeler is adapted to use a mathematical model of biomechanical responses of corneas to perturbations.

45. The system of claim 44 where the mathematical model is based on finite element analysis.

46. The system of claim 44 where the mathematical model is based on representing said model as a portion of a hydrated porous structure.

47. The system of claim 1 where the corneal data is received in a computer system which generates the predictive corneal biomechanical response model.

48. The system of claim 1 further includes means for generating and reading a computer-readable medium containing a data structure for storing corrections for existing algorithms for ablative procedures in cornea surgery, said computer-readable medium comprising an algorithm table containing at least one entry for each of a plurality of existing algorithms for ablative procedures, each said entry containing a plurality of corrections to said existing algorithm, each said plurality of corrections containing corrective specification for said existing algorithm based upon pre-perturbation data of the cornea and post-perturbation data of the cornea.

49. The system of claim 35 wherein the perturbation adapted to create the flap comprises a keratome or a laser.

50. The system of claim 1, where the predictive corneal biomechanical response model is related to a negative pressure change caused by removing cortical tissue.

51. The system of claim 1, where the predictive corneal biomechanical response model is related to an effect of severing one or more lamellae on alternating hard and soft conical layers.

52. The system of claim 42, where the post-operative diagnostic data comprises patient satisfaction data, patient vision acuity data, patient halo effect data, topographic data, pachymetric data, elevation data, total corneal thickness data, corneal curvature data, wave front data, ultrasonic data, intraocular pressure data, and combinations thereof.

53. The system of claim 1 further comprising a computer readable medium storing computer executable instructions operable to perform the system of claim 1.

54. The system of claim 1 where the first corneal data comprises topographic data, pachymetric data, elevation data, corneal thickness data, corneal curvature data, wave front data, intraocular pressure data, peripheral stroma thickness data, ultrasonic data, prior surgical response data, visual acuity data, and combinations thereof.

55. The system of claim 1, where the second corneal data comprises topographic data, pachymetric data, elevation data, corneal thickness data, corneal curvature data, wave front data, intraocular pressure data, peripheral stroma thickness data, ultrasonic data, post-surgical response data, visual acuity data, and combinations thereof.

56. The system of claim 1, where the ablation specifications comprise an ablation size, an ablation shape, an ablation location, an ablation depth, a number of ablations, and combination thereof.

57. The system of claim 1 further includes a data structure in communication with the modeler and/or ablation specification processor, the data structure comprises:
a first field that holds information concerning one or more first corneal measurements taken before one or more of the perturbation;
a second field that holds information concerning one or more second corneal measurements taken after one or more of the perturbation; and
a third field that holds information concerning one or more ablation specifications, where the ablation specifications are derived, at least in part, from at least one of the one or more first corneal measurements, the one or more second corneal measurements, and a predictive biomechanical response model.

58. The system of claim 1 further comprising:
a memory that stores one or more computer executable corneal ablation programs and one or more ophthalmic ablation specifications associated with a refractive ablation procedure to be performed by the laser; and
an adjustment data processing component that computes an adjustment to the one or more computer executable corneal ablation program and/or the one or more ophthalmic ablation specifications based on the first corneal data and the second corneal data as modeled in the predictive corneal biomechanical response model.
